# EUROPEAN PATENT APPLICATION

(11) **EP 4 772 197 A1**
(43) Date of publication of application: **08.07.2026**
(21) Application number: 24859718.9
(22) Date of filing: 26.08.2024
(51) Int. Cl.: A61K 47/65, A61K 38/07, A61K 47/68, A61P 35/00, C07K 2/00, C07K 7/08, C07K 16/18

(54) **TARGETED DRUG CONJUGATE, MEDICINE INCLUDING THE SAME, AND COMPOUND**

(30) Priority: 31.08.2023 JP 2023141861
(71) Applicant: FUJIFILM Corporation, Tokyo 106-8620 (JP)
(72) Inventor: TAMURA, Takashi, Ashigarakami-gun, Kanagawa 258-8577 (JP); WATANABE, Kousuke, Ashigarakami-gun, Kanagawa 258-8577 (JP); TANAKA, Hiroaki, Ashigarakami-gun, Kanagawa 258-8577 (JP)
(74) Representative: Klunker IP Patentanwälte PartG mbB
(86) International application number: PCT/JP2024/030326
(87) International publication number: WO 2025/047686

(57) **Abstract**

Provided are a compound or a salt thereof, including two or more structural moieties M selected from a physiologically active substance moiety and a radioisotope-containing moiety; and a structure represented by General Formula (I); as well as a targeted drug conjugate and a medicine including the same.

In this regard, the above-mentioned compound and a salt do not have a phosphor moiety and have at least one group selected from a specific hydrophilic group Pi.

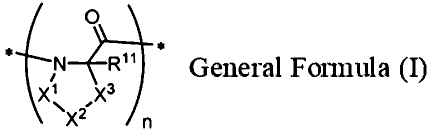

In the formula, X¹ to X³ each represent -O-, -S-, >NR¹, or >CR²R³. R¹ to R³ and R¹¹ each represent a hydrogen atom or a substituent.

n is an integer of 2 or more. * represents a bonding site.

## Description

### BACKGROUND OF THE INVENTION

### 1. Field of the Invention

The present invention relates to a targeted drug conjugate, a medicine including the same, and a compound.

### 2. Description of the Related Art

A targeted drug conjugate is a drug in which a target binding substance that specifically binds to a specific cell that causes a disease (for example, a tumor cell) is conjugated with a physiologically active substance (drug) or the like. The targeted drug conjugate makes it possible for a drug to be delivered to and act on the specific cell with pinpoint accuracy. In other words, it is possible to treat cells that cause a disease with a drug without damaging healthy normal cells. It is said that the use of a targeted drug conjugate makes it possible to enhance therapeutic effects while suppressing side effects with a smaller amount of a drug than in a case where the drug itself is administered orally or intravascularly.

An antibody drug conjugation (ADC) is a targeted drug conjugate that uses an antibody as the target binding substance. So far, MYLOTARG, ZEVALIN, KADCYLA, ADCETRIS, BESPONSA, ENHERTU, AKALUX, POLIVY, PADCEV, and ELAHERE (all of which are trade names) have been approved as ADCs and are actually used in medical settings.

Unlike a drug used in general chemotherapy such as oral administration and intravascular administration, the use of an ADC makes it possible for a drug to act on target cells with pinpoint accuracy. However, in a case where an ADC is used, the amount of drug that can act on the target cells is limited, and thus a drug having high physiological activity is conjugated with an antibody in the ADC. Nevertheless, there is a strong demand for higher therapeutic effects, and as a solution to this, it has been proposed to bind a larger amount of a drug to an antibody. In addition, in a case where it is possible to bind a large amount of a drug to an antibody, even a drug that has a certain degree of low physiological activity and is unsuitable for ADCs may be able to exhibit the desired therapeutic effects specifically on target cells. However, in ADCs approved by the U.S. Food and Drug Administration (FDA), the number of drugs (the number of molecules that exhibit the desired physiological activity) linked to one antibody molecule is limited to about 2 to 8.

On the other hand, a drug generally has high hydrophobicity and, in a case where a conjugate is formed by binding a large number of drugs to an antibody having high hydrophilicity, there is a concern that the drugs may interact with each other in a hydrophobic manner, making the conjugate more susceptible to aggregation.

In relation to a technique of linking a larger amount of a drug to one antibody molecule, for example, JP2020-536847A, JP2021-510700A, and JP2022-526651A describe a polymer structure body which has a phosphoalkyl group or a phosphoalkyl ether group as a repeating structure in a main chain of a polymer structure, and in which the drug is bound to the repeating structure.

In addition, JP2021-506788A describes a polymer structure body which has a structural moiety having a charge such as an ammonium salt as a repeating structure in a main chain of a polymer structure, and in which the drug is bound to the repeating structure.

Furthermore, WO2021/240155A describes a polymer structure body in which a polymer structure in which a repeating structure of an acyclic amino acid and an alkylene glycol are linked to each other is taken as a main chain, and the drug is bound to the repeating structure of an acyclic amino acid.

In addition, although it is not an ADC, a technique of linking a larger amount of a fluorescent compound to one antibody molecule is known. For example, WO2023/032995A describes a compound having two or more phosphor moieties each having equivalent light absorption characteristics, in which the phosphor moieties adjacent to each other are linked through a ring structure derived from proline or the like, and a labeled biological substance using the compound. WO2023/032995A describes that the functioning of the ring structure derived from proline or the like as a rigid linker makes it possible to effectively suppress quenching due to inter- or intramolecular association of the phosphor moieties, thereby obtaining a labeled biological substance that exhibits an excellent fluorescence intensity.

### SUMMARY OF THE INVENTION

An object of the present invention is to provide a targeted drug conjugate capable of retaining a larger amount of a drug while suppressing aggregation, and a medicine including the same. In addition, another object of the present invention is to provide a compound suitable for obtaining the above-mentioned targeted drug conjugate or medicine.

That is, the above-mentioned objects of the present invention have been achieved by the following means.

[1]
A compound or a salt thereof, comprising:
two or more structural moieties M selected from a physiologically active substance moiety and a radioisotope-containing moiety; and
a structure represented by General Formula (I).

In this regard, the above-mentioned compound and a salt do not have a phosphor moiety and have at least one group selected from the following hydrophilic group Pi.

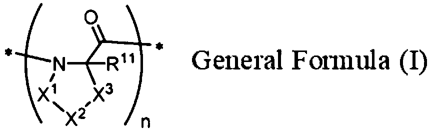

In the formula, X¹ to X³ each represent -O-, -S-, >NR¹, or >CR²R³.

R¹ to R³ and R¹¹ each represent a hydrogen atom, an alkyl group, an alkenyl group, an alkynyl group, an aryl group, a heteroaryl group, an acyl group, -NR⁸R⁹, -OR¹⁰, or a group selected from the following hydrophilic group Pi.

R⁸ to R¹⁰ each represent a hydrogen atom, an alkyl group, an alkenyl group, an alkynyl group, an acyl group, an aryl group, a heteroaryl group, or a group selected from the following hydrophilic group Pi.

n is an integer of 2 or more.

* represents a bonding site.
<Hydrophilic group Pi>
an anionic group, a cationic group, a betaine residue, a polyol residue, a sugar residue, a polyamino acid residue, and a polyalkyleneoxy group.

[2]
The compound or a salt thereof according to [1], which is represented by General Formula (II).

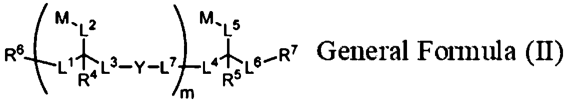

In the formula, R⁴ and R⁵ each represent a hydrogen atom, an alkyl group, an alkenyl group, an alkynyl group, an acyl group, an amino group, a hydroxy group, an alkoxy group, a sulfanyl group, an aryl group, or a heteroaryl group.

R⁶ and R⁷ each represent a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, at least one of R⁶ or R⁷ is a group containing a carboxy group or a group containing a substituent capable of binding to a target binding substance.

Q represents a group selected from the hydrophilic group Pi or a substituent capable of binding to the target binding substance.

L¹, L³, L⁴, L⁶, and L⁷ each represent a single bond or a linking group.

L² and L⁵ each represent a single bond or a physiologically cleavable linker.

In this regard, in a case where L² is a single bond, at least one of L¹ or L³ is a physiologically cleavable linker, and in a case where L⁵ is a single bond, at least one of L⁴ or L⁶ is a physiologically cleavable linker.

M represents the structural moiety M.

Y represents the structure represented by General Formula (I).

m is an integer of 1 or more.

[3]
The compound or a salt thereof according to [2], which is represented by General Formula (III).

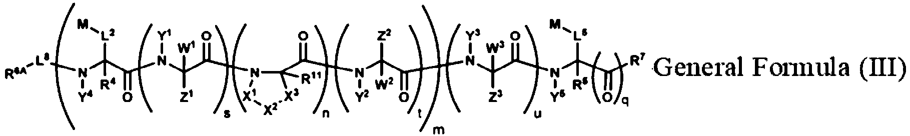

In the formula, Y¹ to Y⁵, Z¹ to Z³, and W¹ to W³ each represent a hydrogen atom, an alkyl group, an alkenyl group, an alkynyl group, an acyl group, an amino group, a hydroxy group, an alkoxy group, a sulfanyl group, an aryl group, a heteroaryl group, or a group selected from the hydrophilic group Pi.

R^{6A} represents a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, R^{6A} is a carboxy group or a substituent capable of binding to a target binding substance and/or R⁷ is a group containing a carboxy group or a group containing a substituent capable of binding to the target binding substance.

R⁴, R⁵, R⁷, R¹¹, L², L⁵, X¹ to X³, M, Q, m, and n have the same definitions as R⁴, R⁵, R⁷, R¹¹, L², L⁵, X¹ to X³, M, Q, m, and n described above.

L⁸ represents a single bond or a linking group.

s, t, and u each represent 0 or an integer of 1 or more.

q is an integer of 0 or 1.

[4]
The compound or a salt thereof according to [1], which is represented by General Formula (IV).

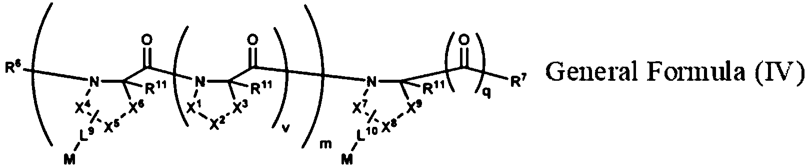

In the formula, X⁴ to X⁹ each represent -O-, -S-, >NR¹, or >CR²R³.

In this regard, at least one of X⁴, X⁵, or X⁶ is >N-L⁹-M or >C(R²)-L⁹-M, and at least one of X⁷, X⁸, or X⁹ is >N-L¹⁰-M or >C(R²)-L¹⁰-M.

R¹ to R³ which are neither -L⁹-M nor -L¹⁰-M have the same definitions as R¹ to R³ described above.

L⁹ and L¹⁰ each represent a physiologically cleavable linker.

R⁶ and R⁷ each represent a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, at least one of R⁶ or R⁷ is a group containing a carboxy group or a group containing a substituent capable of binding to a target binding substance.

Q represents a group selected from the hydrophilic group Pi or a substituent capable of binding to the target binding substance.

m is an integer of 1 or more, q is an integer of 0 or 1, and v is 0 or an integer of 1 or more.

R¹¹, X¹ to X³, and M have the same definitions as R¹¹, X¹ to X³, and M described above.

[5]
The compound or a salt thereof according to [4], which is represented by General Formula (V).

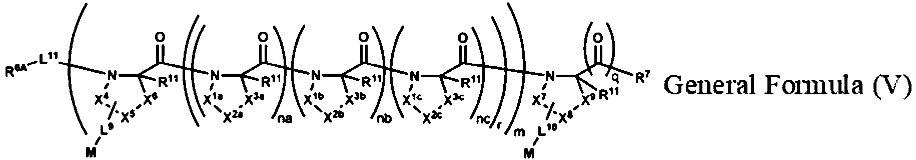

In the formula, X^{1a}, X^{1b}, X^{1c}, X^{2a}, X^{2b}, X^{2c}, X^{3a}, X^{3b}, and X^{3c} each represent -O-, -S-, >NR¹, or >CR²R³.

L¹¹ represents a single bond or a linking group.

R^{6A} represents a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, R^{6A} is a carboxy group or a substituent capable of binding to a target binding substance and/or R⁷ is a group containing a carboxy group or a group containing a substituent capable of binding to the target binding substance.

R¹ to R³, R⁷, R¹¹, X⁴ to X⁹, L⁹, L¹⁰, M, Q, m, and q have the same definitions as R¹ to R³, R⁷, R¹¹, X⁴ to X⁹, L⁹, L¹⁰, M, Q, m, and q described above.

na, nb, and nc are each 0 or an integer of 1 or more. r is an integer of 1 or more.

[6]
The compound or a salt thereof according to any one of [1] to [5], which has three to six structural moieties M.

[7]
A targeted drug conjugate comprising:
the compound or a salt thereof according to any one of [1] to [6]; and
a target binding substance,
in which the compound or a salt thereof is bound to the target binding substance.

[8]
The targeted drug conjugate according to [7], in which the target binding substance is an antibody.

[9]
The targeted drug conjugate according to [7] or [8], in which the number of the structural moieties M in one molecule of the targeted drug conjugate is 8 or more.

[10]
A medicine comprising:
the targeted drug conjugate according to any one of [7] to [9].

The targeted drug conjugate and the medicine including the same according to the embodiment of the present invention can retain a larger amount of a drug while suppressing aggregation. In addition, the compound according to the embodiment of the present invention makes it possible to suitably obtain the above-mentioned targeted drug conjugate and the above-mentioned medicine including the same.

### DESCRIPTION OF THE PREFERRED EMBODIMENTS

In the present invention, in a case where there are a plurality of substituents, linking groups, structural units, or the like (hereinafter, referred to as substituents or the like), which are represented by a specific reference numeral or formula, or in a case where a plurality of substituents or the like are defined at the same time, the substituents or the like may be the same as or different from each other, unless otherwise specified. The same applies to the definition of the number of substituents or the like. In a case where a plurality of substituents or the like come close to each other (particularly in a case where a plurality of substituents or the like are adjacent to each other), the substituents or the like may be linked to each other to form a ring unless otherwise specified. In addition, unless otherwise specified, rings, for example, an alicyclic ring, an aromatic ring, and a heterocyclic ring may be further fused to form a fused ring.

In describing a group (atomic group) in the present invention, in a case where a description of substitution and unsubstitution is not provided, the description includes a group (atomic group) having a substituent as well as a group (atomic group) having no substituent. For example, the term "alkyl group" includes not only an alkyl group having no substituent (unsubstituted alkyl group) but also an alkyl group having a substituent (substituted alkyl group). The same applies to a divalent linking group such as an alkylene group. Preferred examples of the optional substituent contained in such a group (atomic group) having a substituent include a substituent selected from the substituent group T which will be described later.

In the present invention, in a case where the number of carbon atoms in a certain group is defined, the number of carbon atoms refers to the number of carbon atoms in the entire group unless otherwise specified in the present invention or the present specification. In other words, in a case where this group is in a form in which it further has a substituent, the number of carbon atoms refers to the total number of carbon atoms including the substituent.

In addition, in the present invention, the numerical range represented by using "to" means a range including the numerical values before and after "to" as the lower limit value and the upper limit value, respectively.

In addition, the chemical structural formula in the present invention may be described by a simplified structural formula in which a hydrogen atom is omitted.

In the present invention, the term "alkyl group" is used to include a linear alkyl group, a branched alkyl group, and a cycloalkyl group. The same applies to groups that can adopt other cyclic structures (an alkenyl group, an alkynyl group, and the like), and groups containing groups that can adopt other cyclic structures (an alkyl group, an alkenyl group, an alkynyl group, and the like) (an alkoxy group, an alkylthio group, an alkenyloxy group, and the like). In a case where the group can form a cyclic skeleton, the lower limit of the number of atoms in the group forming the cyclic skeleton is at least 3 or more, preferably 4 or more, and more preferably 5 or more, regardless of the lower limit of the number of atoms specifically described below for the group that can adopt this structure.

In the present invention, the "alkyl group" which does not have a cyclic structure preferably has 1 to 20 carbon atoms, more preferably 1 to 12 carbon atoms, still more preferably 1 to 8 carbon atoms, even still more preferably 1 to 6 carbon atoms, and particularly preferably 1 to 3 carbon atoms.

In the present invention, the "cycloalkyl group", which is an alkyl group having a cyclic structure, preferably has 3 to 20 carbon atoms, more preferably 3 to 12 carbon atoms, still more preferably 3 to 8 carbon atoms, even still more preferably 3 to 6 carbon atoms, and particularly preferably 3 carbon atoms.

In the present invention, the "alkenyl group" which does not have a cyclic structure preferably has 2 to 20 carbon atoms, more preferably 2 to 12 carbon atoms, still more preferably 2 to 6 carbon atoms, and even still more preferably 2 to 4 carbon atoms.

In the present invention, the "cycloalkenyl group", which is an alkenyl group having a cyclic structure, preferably has 4 to 20 carbon atoms, more preferably 4 to 12 carbon atoms, still more preferably 4 to 8 carbon atoms, and even still more preferably 4 to 6 carbon atoms.

In the present invention, the "alkyl group" and the "alkenyl group" are each preferably an "alkyl group" which does not have a cyclic structure and an "alkenyl group" which does not have a cyclic structure.

In the present invention, the "alkynyl group" which does not have a cyclic structure preferably has 2 to 20 carbon atoms, more preferably 2 to 12 carbon atoms, still more preferably 2 to 6 carbon atoms, and even still more preferably 2 to 4 carbon atoms.

In the present invention, the "aryl group" may be a monocyclic group or a fused ring group. In a case of a fused ring group, it is preferably a fused ring group having 2 to 6 rings. The ring constituting the fused ring is preferably a 6- or 8-membered ring, and more preferably a 6-membered ring. The monocyclic ring is preferably a 6-membered ring. The aryl group preferably has 6 to 40 carbon atoms, more preferably 6 to 30 carbon atoms, still more preferably 6 to 26 carbon atoms, and particularly preferably 6 to 10 carbon atoms.

In the present invention, the term "heterocyclic group" includes an "aliphatic heterocyclic group" and a "heteroaryl group". The "heterocyclic group" may be a group having at least one nitrogen atom, oxygen atom, sulfur atom, phosphorus atom, silicon atom, or selenium atom as a ring-constituting atom, may be a monocyclic group or may be a fused ring group. In a case of a fused ring group, it is preferably a fused ring group having 2 to 6 rings. The number of ring members in the ring in the monocyclic group and the ring constituting the fused ring group is preferably 5 to 7, and more preferably 5 or 6. The number of carbon atoms in the heterocyclic group is preferably 2 to 40, and more preferably 2 to 20.

In the present invention, the "alkoxy group" preferably has 1 to 20 carbon atoms, more preferably 1 to 12 carbon atoms, still more preferably 1 to 8 carbon atoms, even still more preferably 1 to 6 carbon atoms, and particularly preferably 1 to 3 carbon atoms.

In the present invention, examples of the "amino group" include an unsubstituted amino group (-NH₂), as well as a mono- or di-substituted amino group substituted with a group selected from an alkyl group, an alkenyl group, an alkynyl group, a cycloalkyl group, a cycloalkenyl group, an aryl group, and a heterocyclic ring. The above-mentioned groups (an alkyl group, an alkenyl group, an alkynyl group, a cycloalkyl group, a cycloalkenyl group, an aryl group, and a heterocyclic group) for substituting an unsubstituted amino group have the same meanings as the above-mentioned groups (an alkyl group, an alkenyl group, an alkynyl group, a cycloalkyl group, a cycloalkenyl group, an aryl group, and a heterocyclic group). The number of carbon atoms in the amino group is preferably 0 to 20, more preferably 0 to 12, still more preferably 0 to 8, even still more preferably 0 to 6, and particularly preferably 0 to 3.

In the present invention, examples of the "acyl group" include -C(=O)H, an alkylcarbonyl group, a cycloalkylcarbonyl group, an arylcarbonyl group, and a heterocyclic carbonyl group. The above-mentioned groups (an alkyl group, a cycloalkyl group, an aryl group, and a heterocyclic group) for substituting a carbonyl group have the same meanings as the above-mentioned groups (an alkyl group, a cycloalkyl group, an aryl group, and a heterocyclic group). The number of carbon atoms in the acyl group is preferably 1 to 20, and more preferably 2 to 15.

In the present invention, the "alkylene group, alkenylene group, alkynylene group, arylene group, and heteroarylene group" that can constitute the "linking group" have the same meanings as the groups obtained by further removing one hydrogen atom from the above-mentioned "alkyl group, alkenyl group, alkynyl group, aryl group, and heteroaryl group", and the same applies to the preferred ones thereof.

In the present invention, the "physiologically cleavable linker" may be any molecular linkage that can be split or separated by a defined method to yield two or more individual molecules while being present in an in vivo or in vitro environment of an organism or cellular system, preferred examples of which include an amide bond, an ester bond, a disulfide bond, a thioester bond, a thioamide bond, a urea bond, a urethane bond, and a thiourea bond; a linker containing a divalent linking group obtained from hydrazone, imine, acetal, aminal, phosphotriester, diester, β-glucuronide, vinyl ether, dialkyl or diaryl dialkoxysilane, β-thiopropionate, glycine-glycine-phenylalanine, glycine-glycine-phenylalanine-glycine, valine-citrulline, p-aminobenzyl alcohol or p-hydroxybenzyl alcohol structure (hereinafter, referred to as a divalent linking group LL); and a linker containing a linking group formed by combining two or more of these divalent linking groups LL (which may be directly bonded to each other or may be linked through a structure other than the linking group LL). The structure other than the divalent linking group LL contained in the linker is not particularly limited, and examples thereof include a linking group such as an alkylene group.

In the present invention, the "anionic group" may be any group having an anion. Examples of such an anionic group include a carboxy group, a phosphono group (a phosphonate group, -PO(OH)₂), a phosphonooxy group (a phosphate group, -OPO(OH)₂), and a sulfo group, among which a phosphono group, a phosphonooxy group, or a sulfo group is preferable, and a phosphonooxy group or a sulfo group is more preferable.

The anionic group may have an ionic structure formed by dissociating a hydrogen ion, or may have a salt structure. In a case where the anionic group has a salt structure, the description of the salt in the salt allowed as a medicinal product which will be described later can be preferably applied.

In the present invention, the "cationic group" may be any group having a cation. Examples of such a cationic group include a group having a quaternary ammonium ion (an ammonio group), a group having a tertiary sulfonium ion (a sulfonio group), and a group having a quaternary phosphonium ion (a phosphonio group), among which a group having a quaternary ammonium ion is preferable. Preferred examples of the substituent that N⁺ has in the group having a quaternary ammonium ion, the substituent that S⁺ has in the group having a tertiary sulfonium ion, and the substituent that P⁺ has in the group having a quaternary phosphonium ion include an alkyl group and an aryl group. It is more preferable that all the substituents that N⁺, S⁺, and P⁺ have are alkyl groups.

The cationic group may have a salt structure in addition to an ionic structure. Examples of monovalent or polyvalent anions in a case where the cationic group has a salt structure include halide ions such as F⁻ and Cl⁻, and monovalent or polyvalent organic anions such as BF₄⁻, PF₆⁻, and a bis(trifluoromethylsulfonyl)imide ion.

In the present invention, the "betaine residue" means a group obtained by removing one hydrogen atom from a compound having a betaine structure. The compound having a betaine structure may be any compound containing an anionic group and a cationic group in the same molecule, and is preferably a compound containing, in the same molecule, at least one anionic group of a carboxy group, a phosphono group, a phosphonooxy group, or a sulfo group, and at least one cationic group of an ammonio group, a sulfonio group, or a phosphonio group.

A group obtained by removing one hydrogen atom from a compound that does not contain an anionic group and a cationic group in the same molecule does not fall under the category of a betaine residue, and is classified as the above-mentioned anionic group having a salt structure or the above-mentioned cationic group having a salt structure.

In the present invention, the "polyol residue" means a group obtained by removing one hydrogen atom from a polyol compound having two or more hydroxy groups in a molecule, which does not fall under either a sugar residue or a polyalkyleneoxy group which will be described later. The polyol residue may be a chain-like group or a group having a cyclic structure and examples thereof include a cyclodextrin residue.

In the present invention, the "sugar residue" means a group obtained by removing one hydrogen atom from a sugar compound. The sugar compound may be any of a monosaccharide, a polysaccharide in which two or more sugars are bonded, a sugar alcohol, a chemically modified sugar in which epichlorohydrin or the like is copolymerized with a sugar, or the like.

Among the polyol residues, one that falls under the category of a sugar residue is classified as the sugar residue.

Examples of the sugar residue include groups obtained by removing one hydrogen atom from sugar compounds such as glucose, sucrose, maltose, lactose, trehalose, ribitol, sorbitol, mannitol, maltitol, lactitol, xylitol, fruit sugar (fructose), 1-kestose, nystose trihydrate, fucose, dulcitol, galactooligosaccharide, 4'-galactosyl lactose, isomalto-oligosaccharide, lactulose, palatinit, palatinose monohydrate, raffinose pentahydrate, arabinose, dihydroxyacetone dimer, galactose, glyceryl aldehyde dimer, mannose, ribose, xylose, lactosyl fructoside, erythritol, dulcoside A, isosteviol, rebaudioside A, rebaudioside B, rebaudioside C, rebaudioside D, rebaudioside F, rubusoside, stevioside, 1-deoxynojirimycin, fucoidan, rhamnose, threose, arabinose, lyxose, allose, altrose, gulose, idose, talose, 1,3-dihydroxyacetone, erythrose, xylulose, ribulose, psicose, sorbose, tagatose, polysucrose, and fructooligosaccharide.

In the present invention, the "polyamino acid residue" means a group obtained by removing one hydrogen atom from an amino acid compound or a polyamino acid compound in which two or more amino acids are bonded.

In the present invention, the "polyalkyleneoxy group" may be a group represented by -(O-L)_{g}R^{E} or -(L-O)_{g}R^{E}.

Among the polyol residues, one that falls under the category of a polyalkyleneoxy group is classified as the polyalkyleneoxy group.

L represents the above-mentioned alkylene group, and the number of carbon atoms in the alkylene group is preferably 2 to 4, more preferably 2 or 3, and still more preferably 2, and the number of carbon atoms contained in the shortest chain that links the two carbon atoms which are bonding sites of the alkylene group is preferably 0 to 2, more preferably 0 or 1, and still more preferably 0. That is, L is most preferably an ethylene group.

g means an average repetition number (also simply referred to as a repetition number), and is preferably 1 to 24, more preferably 1 to 12, and still more preferably 4 to 12. Even in a case where the repetition number is small, for example, even in a case where g is 1, it is possible to exhibit an appropriate hydrophilicity and an appropriate excluded volume effect.

R^{E} represents a hydrogen atom or an alkyl group. For the alkyl group which can be adopted as R^{E}, the description of the alkyl group described above can be preferably applied, and above all, an alkyl group having 1 to 3 carbon atoms is preferable. The alkyl group which can be adopted as R^{E} may have a substituent.

In addition, in the present invention, a structure represented by General Formula (I) which will be given later means that n (n is an integer of 2 or more) pieces of structures represented by General Formula (i) are connected. In this case, the n pieces of structures represented by General Formulae (i) may be the same as or different from each other. X¹ to X³, and R¹¹ in General Formula (i) have the same definitions as X¹ to X³, and R¹¹ in General Formula (I) which will be given later. The same applies to a structure parenthesized in ( )ₘ, a structure parenthesized in ( )ₙₐ, a structure parenthesized in ( )_{nb}, a structure parenthesized in ( )_{nc}, a structure parenthesized in ( )ᵣ, a structure parenthesized in ( )ₛ, a structure parenthesized in ( )ₜ, a structure parenthesized in ( )ᵤ, and a structure parenthesized in ( )ᵥ, where m pieces of structures may be the same as or different from each other, na pieces of structures may be the same as or different from each other, nb pieces of structures may be the same as or different from each other, nc pieces of structures may be the same as or different from each other, r pieces of structures may be the same as or different from each other, s pieces of structures may be the same as or different from each other, t pieces of structures may be the same as or different from each other, u pieces of structures may be the same as or different from each other, and v pieces of structures may be the same as or different from each other.

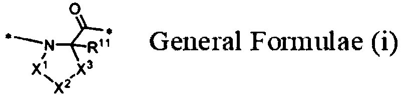

Examples of the salt of the compound according to the embodiment of the present invention include salts in basic groups such as an amino group, and salts in acidic groups such as a hydroxy group, a carboxy group, a phosphono group, a phosphonooxy group, and a sulfo group, which are commonly known.

Examples of the salts in basic groups include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; salts with sulfonic acids such as methanesulfonic acid, benzenesulfonic acid, p-toluenesulfonic acid, mesitylene sulfonic acid, and naphthalene sulfonic acid; and salts with phosphoric acid or the like.

Examples of the salts in acidic groups include salts with alkali metals such as sodium and potassium; salts with alkaline earth metals such as calcium and magnesium; ammonium salts; and salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine. That is, in the present invention, the acidic group is used to include a salt type group.

Among the above salts, preferred examples of the salt include a salt that is acceptable as a medicinal product and a pharmacologically acceptable salt.

In a case where the compound according to the embodiment of the present invention has a salt structure, the type of the salt may be one type, two or more types of the salts may be present together, a salt type and a group having a free acid structure may be present together in a compound, and a compound having a salt structure and a compound having a free acid structure may be present together.

In the compound according to the embodiment of the present invention or a salt thereof, in a case where a stereoisomer such as an optical isomer, a geometrical isomer, or a rotational isomer, or a tautomer is present, the present invention includes these isomers, as well as an anhydride, a solvate, a hydrate, and various forms of crystals.

The compound according to the embodiment of the present invention is meant to include a compound in which the structure has been partially modified within a range which does not impair the effect of the present invention.

The compound according to the embodiment of the present invention or a salt thereof (hereinafter, collectively referred to as the compound according to the embodiment of the present invention) is a compound or a salt thereof, which includes two or more structural moieties M selected from a physiologically active substance moiety and a radioisotope-containing moiety, and a structure represented by General Formula (I) which will be described later. Although the details of why the compound according to the embodiment of the present invention makes it possible to obtain a targeted drug conjugate in which a larger amount of a drug can be retained while suppressing aggregation, as the structural moiety M selected from a physiologically active substance moiety and a radioisotope-containing moiety, are not clear, it is thought that the reason is as follows.

The compound according to the embodiment of the present invention is a compound including two or more structural moieties M selected from a physiologically active substance moiety and a radioisotope-containing moiety, and a structure represented by General Formula (I) which will be described later, and has at least one group selected from the hydrophilic group Pi which will be described later. Therefore, it is considered that, even in a case where two or more structural moieties M exhibiting hydrophobicity are linked, the obtained targeted drug conjugate or medicine can retain a larger amount of a drug while suppressing aggregation, and can stably transport the drug to the desired target cells. Therefore, the compound according to the embodiment of the present invention is expected to be applicable to a variety of therapeutic methods, such as the use of a drug having low physiological activity for ADCs, which have not previously been possible to use in ADCs.

Furthermore, since the compound according to the embodiment of the present invention includes, as the structure represented by General Formula (I), a very stable structure that is inhibited from being decomposed by an enzyme contained in the blood, consisting of a repeating unit (where the repetition number n is an integer of 2 or more) containing a nitrogen-containing saturated 5-membered ring such as a ring structure derived from proline, which is one of amino acids that constitute a protein, it is considered that it is possible to suppress the problem of the obtained targeted drug conjugate being decomposed by an enzyme in the blood, and it is possible to reduce the possibility of normal tissues being exposed to the drug due to decomposition and cleavage of the targeted drug conjugate before reaching the desired target cells, and then release of the drug from the structural moiety M, which enables the drug to be transported more stably in the blood (the stability in the blood is also excellent).

Hereinafter, the compound according to the embodiment of the present invention will be described in more detail.

### <Compound according to embodiment of present invention>

The compound according to the embodiment of the present invention is a compound including two or more structural moieties M selected from a physiologically active substance moiety and a radioisotope-containing moiety, and a structure represented by General Formula (I). In this regard, the compound according to the embodiment of the present invention does not have a phosphor moiety and has at least one group selected from the hydrophilic group Pi which will be described later.

The compound according to the embodiment of the present invention may be a compound classified as a polymer or an oligomer.

The structural moiety M may be contained in the compound according to the embodiment of the present invention in a state where the structural moiety M is bonded to a bonding site * in General Formula (I) directly or through a linking group as a structure separate from the structure represented by General Formula (I), or may be contained in the compound according to the embodiment of the present invention in a state where the structural moiety M is bonded to any site of X¹, X², or X³ in the structure represented by General Formula (I) through a linking group.

Examples of the form in which the structural moiety M is bonded to the bonding site * in General Formula (I) directly or through a linking group include a compound represented by General Formula (II) or (III) which will be described later. In addition, examples of the form in which the structural moiety M is bonded to any site of X¹, X², or X³ in the structure represented by General Formula (I) through a linking group include a compound represented by General Formula (IV) or (V) which will be described later. In the compound represented by General Formula (IV) or (V) which will be described later, in a case where v in General Formula (IV) is an integer of 2 or more, and in a case where a combination of na + nb + nc and r in General Formula (V) is a combination in which one is an integer of 1 or more and the other is an integer of 2 or more, the structural moiety M may be in a form of being bonded to the bonding site * in General Formula (I) through a linking group.

The compound according to the embodiment of the present invention is preferably a compound in which structural moieties M adjacent to each other are linked through a structure represented by General Formula (I), that is, a compound in which two structures having structural moieties M as substituents are linked through a group containing a structure represented by General Formula (I), and more preferably a compound in which structural moieties M adjacent to each other are bonded to a bonding site * in General Formula (I) through a linking group. In the compound in which structural moieties M adjacent to each other are bonded to the bonding site * in General Formula (I) through a linking group, X¹ to X³ and R¹¹ in General Formula (I) are not groups containing the structural moiety M.

### (Structural moiety M)

The compound according to the embodiment of the present invention includes two or more structural moieties M selected from a physiologically active substance moiety and a radioisotope-containing moiety.

It is noted that the two or more structural moieties M selected from a physiologically active substance moiety and a radioisotope-containing moiety may be the same as or different from each other.

The physiologically active substance moiety which can be adopted as the structural moiety M can be used without particular limitation as long as it is a structural moiety consisting of a drug (physiologically active substance) or a prodrug moiety which will be described later.

Examples of the physiologically active substance moiety include an anti-tumor agent, an enediyne anti-tumor antibiotic, a maytansinoid, a topoisomerase inhibitor, a non-steroidal anti-inflammatory drug (NSAID), a kinase inhibitor, an anthracycline, an epidermal growth factor receptor (EGFR) inhibitor, an alkylating agent, an anticancer drug, an antibiotic, an antifungal drug, an anthelmintic drug, and an antiviral drug, more specific examples of which include those described in paragraphs [0105] to [0117] of JP2022-526651A, which relate to biologically active moieties such as an anti-tumor agent, an enediyne anti-tumor antibiotic, a maytansinoid, a topoisomerase inhibitor, a non-steroidal anti-inflammatory drug (NSAID), a kinase inhibitor, an anthracycline, an epidermal growth factor receptor (EGFR) inhibitor, an alkylating agent, and an anticancer drug, and those described in paragraphs [0185] to [0207] of JP2022-526651A, which relate to biologically active moieties such as an antibiotic, an antifungal drug, an anthelmintic drug, and an antiviral drug.

In addition to the above, examples of the physiologically active substance include a vitamin, a coenzyme, a hormone, an antibiotic, a neurotransmitter, and a cytokine. More specific examples of other physiologically active substances include calicheamicin, doxorubicin, daunorubicin, mitomycin C, bleomycin, cyclocytidine, vincristine, vinblastine, methotrexate, cisplatin or a derivative thereof, auristatin or a derivative thereof, maytansine or a derivative thereof, taxol or a derivative thereof, and camptothecin or a derivative thereof, which are described in paragraph [0095] of JP2021-020956A, to which the descriptions in paragraphs [0095] to [0099] of JP2021-020956A can be applied.

Any structural moiety can be used without any particular limitation as the prodrug moiety as long as it is a structural moiety consisting of a compound that is metabolized in vivo to be converted into a drug (physiologically active substance). As for the prodrug moiety, for example, the description in paragraph [0003] of JP2020-105187A (a prodrug form of 2-pyrrolinodoxorubicin) can be applied.

Any structural moiety can be used without any particular limitation as the radioisotope-containing moiety which can be adopted as the structural moiety M as long as it is a structural moiety that contains a radioisotope (a metal ion or a metal complex) that can be used in the medical field and can be used for therapy or diagnosis. Examples of the radioisotope include a nuclide that emits a gamma ray, a positron, an alpha ray, or a beta ray, examples of which include, but are not limited to, ⁶⁷Ga, ⁶⁸Ga, iodine-131, indium-111, yttrium-90, lutetium-177, and copper-64. The descriptions in paragraphs [0080] to [0086] of WO2016/153054A and paragraph [0225] of JP2021-011483A can be applied. Examples of the structural moiety containing a radioisotope include a structural moiety in which the radioisotope is bonded or coordinated to a nitrogen atom of an amino group or a tertiary amine, a sulfanyl group, an aryl group, a heteroaryl group, or the like. Examples of the structural moiety in which a nitrogen atom of a tertiary amine is coordinated to the radioisotope include a structural moiety in which 1,4,7,10-tetraazacyclododecane-1,4,7,10-tetraacetic acid (DOTA) or the like is coordinated to the radioisotope to form a complex, and examples of the structural moiety in which a sulfanyl group is coordinated to the radioisotope include a structural moiety consisting of a complex such as copper (II) diacetylbis(N(4)-methylthiosemicarbazone).

In the compound according to the embodiment of the present invention, the number of the structural moieties M is 2 or more, preferably 2 to 8, and more preferably 3 to 6.

### (Structure represented by General Formula (I))

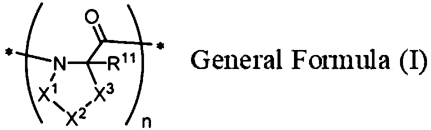

In the formula, X¹ to X³ each represent -O-, -S-, >NR¹, or >CR²R³.

R¹ to R³ and R¹¹ each represent a hydrogen atom, an alkyl group, an alkenyl group, an alkynyl group, an aryl group, a heteroaryl group, an acyl group, -NR⁸R⁹, -OR¹⁰, or a group selected from the following hydrophilic group Pi.

R⁸ to R¹⁰ each represent a hydrogen atom, an alkyl group, an alkenyl group, an alkynyl group, an acyl group, an aryl group, a heteroaryl group, or a group selected from the following hydrophilic group Pi.

n is an integer of 2 or more.

* represents a bonding site.

<Hydrophilic group Pi>
an anionic group, a cationic group, a betaine residue, a polyol residue, a sugar residue, a polyamino acid residue, and a polyalkyleneoxy group.

In the present invention, in consideration of stereoisomers, the structure represented by General Formula (I) is preferably a structure represented by any one of General Formula (IA) or (IB). It is noted that X¹ to X³, R¹¹, and n in the following general formulae have the same definitions as X¹ to X³, R¹¹, and n in General Formula (I).

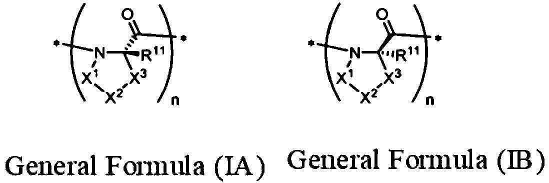

X¹ to X³ each represent -O-, -S-, >NR¹, or >CR²R³, and R¹ to R³ and R¹¹ each represent a hydrogen atom, an alkyl group, an alkenyl group, an alkynyl group, an aryl group, a heteroaryl group, an acyl group, -NR⁸R⁹, -OR¹⁰, or a group selected from the hydrophilic group Pi.

The preferred ranges of the alkyl group, alkenyl group, alkynyl group, aryl group, heteroaryl group, and acyl group, which can be adopted as R¹ to R³ and R¹¹, and the anionic group, cationic group, betaine residue, polyol residue, sugar residue, polyamino acid residue, and polyalkyleneoxy group in the hydrophilic group Pi are as described above.

The alkyl group, alkenyl group, alkynyl group, aryl group, heteroaryl group, and acyl group, which can be adopted as R¹ to R³ and R¹¹, and the betaine residue, polyol residue, sugar residue, polyamino acid residue, and polyalkyleneoxy group in the hydrophilic group Pi each may be unsubstituted or each may have a substituent.

Examples of the substituent which may be contained in the alkyl group, alkenyl group, alkynyl group, aryl group, heteroaryl group, and acyl group in R¹ to R³ and R¹¹, and the betaine residue, polyol residue, sugar residue, polyamino acid residue, and polyalkyleneoxy group in the hydrophilic group Pi include the substituents in the substituent group T which will be described later. For example, a halogen atom or a group selected from the hydrophilic group Pi is preferable.

As described above, any one of X¹, X², or X³ may be >NR¹ or >CR²R³, and any one of R¹ to R³ may be a substituent containing the structural moiety M. Examples of such a compound include a compound represented by General Formula (IV) or (V) which will be described later.

In -NR⁸R⁹ and -OR¹⁰ which can be adopted as R¹ to R³ and R¹¹, R⁸ to R¹⁰ each represent a hydrogen atom, an alkyl group, an alkenyl group, an alkynyl group, an aryl group, a heteroaryl group, an acyl group, or a group selected from the hydrophilic group Pi.

The preferred ranges of the alkyl group, alkenyl group, alkynyl group, aryl group, heteroaryl group, and acyl group, which can be adopted as R⁸ to R¹⁰, and the anionic group, cationic group, betaine residue, polyol residue, sugar residue, polyamino acid residue, and polyalkyleneoxy group in the hydrophilic group Pi are as described above.

The alkyl group, alkenyl group, alkynyl group, aryl group, heteroaryl group, and acyl group, which can be adopted as R⁸ to R¹⁰, and the anionic group, cationic group, betaine residue, polyol residue, sugar residue, polyamino acid residue, and polyalkyleneoxy group in the hydrophilic group Pi each may be unsubstituted or each may have a substituent.

Examples of the substituent which may be contained in the alkyl group, alkenyl group, alkynyl group, aryl group, heteroaryl group, and acyl group in R⁸ to R¹⁰, and the anionic group, cationic group, betaine residue, polyol residue, sugar residue, polyamino acid residue, and polyalkyleneoxy group in the hydrophilic group Pi include the substituents in the substituent group T which will be described later. For example, the substituent is preferably a halogen atom, a carbamoyl group, an acylamino group, an alkoxy group (preferably an alkoxy group having a group selected from the hydrophilic group Pi), a heteroaryl group, or a group selected from the hydrophilic group Pi, and more preferably a carbamoyl group, an acylamino group, an alkoxy group (preferably an alkoxy group having a group selected from the hydrophilic group Pi), a heteroaryl group, or a group selected from the hydrophilic group Pi.

R¹ is preferably a hydrogen atom, an alkyl group, -NR⁸R⁹, -OR¹⁰, or a group selected from the hydrophilic group Pi.

It is preferable that R² and R³ are each a hydrogen atom, -NR⁸R⁹, -OR¹⁰, or a group selected from the hydrophilic group Pi, and it is more preferable that R² is a hydrogen atom, -NR⁸R⁹, -OR¹⁰, or a group selected from the hydrophilic group Pi, and R³ is a hydrogen atom.

R¹¹ is preferably a hydrogen atom, an alkyl group, -NR⁸R⁹, -OR¹⁰, or a group selected from the hydrophilic group Pi, and more preferably a hydrogen atom.

R⁸ to R¹⁰ are each preferably a hydrogen atom, an alkyl group, or an acyl group. The alkyl group may be an alkyl group having at least one of the groups selected from the hydrophilic group Pi, and the acyl group may be an acyl group containing at least one of the groups selected from the hydrophilic group Pi.

The acyl group containing at least one of the groups selected from the hydrophilic group Pi may preferably be an acyl group having at least one of the groups selected from the hydrophilic group Pi, or an acyl group having at least one of the groups selected from the hydrophilic group Pi and substituted with an alkoxy group or a heteroaryl group. The alkyl group and acyl group may have a substituent other than the group selected from the hydrophilic group Pi, and examples of the substituent which may be contained include the substituents in the substituent group T which will be described later, among which, for example, a carbamoyl group or an acylamino group is preferable.

The above-mentioned -(O-L)_{g}R^{E} and -(L-O)_{g}R^{E}, which are polyalkyleneoxy groups, are included as a divalent group obtained by removing one hydrogen atom from a hydrogen atom or a substituent which can be adopted as R^{E}, and a carboxy group, a sulfo group, a phosphono group, a betaine residue, a polyol residue, a sugar residue, or a polyamino acid residue may be further bonded thereto. Examples of the divalent group obtained by removing one hydrogen atom from a hydrogen atom or a substituent which can be adopted as R^{E}, from -(O-L)_{g}R^{E} or -(L-O)_{g}R^{E}, include -(O-L)_{g}-, -(O-L)_{g}-NHCO-, -(O-L)_{g}-CONH-, -(O-L)_{g}-CONH alkylene-, -(O-L)_{g}-NHCO alkylene-, -(L-O)_{g}-, -(L-O)_{g}-NHCO-, -(L-O)_{g}-CONH-, -(L-O)_{g}-CONH alkylene-, and -(L-O)_{g}-NHCO alkylene-.

With regard to X¹ to X³, it is preferable that at least any one of X¹, X², or X³ is >CR²R³, and it is more preferable that at least two of X¹ to X³ are >CR²R³ and the remaining one is -O-, -S-, or >CR²R³.

As described above, in a case where a large number of drugs are bound to a target binding substance such as an antibody that exhibits high hydrophilicity to form a targeted drug conjugate, there is a concern that drugs, which generally exhibit high hydrophobicity, may interact with each other in a hydrophobic manner, making the conjugate more susceptible to aggregation. From the viewpoint of eliminating this concern, it is preferable that the structure represented by General Formula (I) includes a structure in which X¹ to X³ are >CR²R³. That is, the phrase "the structure represented by General Formula (I) includes a structure in which X¹ to X³ are >CR²R³" means that all of X¹ to X³ are >CR²R³ in at least one structure represented by General Formula (i) among n pieces of the consecutive structures represented by General Formula (i) described above.

Of the structures represented by General Formula (I), a proportion of the number of the structures in which X¹ to X³ are >CR²R³ is preferably 30% or more, more preferably 60% or more, and still more preferably 80% or more. The upper limit value of the proportion is not particularly limited and can be 100% or less. It is also preferable that all of the structures represented by General Formula (I) are the structures in which X¹ to X³ are >CR²R³.

From the viewpoint of eliminating the concern that the above-mentioned conjugate may be prone to aggregation, it is preferable that at least one R² in the structure in which X¹ to X³ are >CR²R³ is -NR⁸R⁹, -OR¹⁰, or a group selected from the hydrophilic group Pi; and from the viewpoint of imparting sufficient hydrophilicity to the targeted drug conjugate and further suppressing aggregation of the conjugate, it is more preferable that at least one R² in the structure in which X¹ to X³ are >CR²R³ is -NR⁸R⁹, -OR¹⁰, or a group selected from the hydrophilic group Pi and at least one of R⁸, R⁹, or R¹⁰ is a group containing at least one of the groups selected from the hydrophilic group Pi.

That is, the phrase "at least one R² in the structure in which X¹ to X³ are >CR²R³ is -NR⁸R⁹, -OR¹⁰, or a group selected from the hydrophilic group Pi and at least one of R⁸, R⁹, or R¹⁰ is a group containing at least one of the groups selected from the hydrophilic group Pi" means that, in a case where R² is -NR⁸R⁹, at least one of R⁸ or R⁹ is a group containing at least one of the groups selected from the hydrophilic group Pi, and in a case where R² is -OR¹⁰, R¹⁰ is a group containing at least one of the groups selected from the hydrophilic group Pi.

In addition, R¹⁰ being a group containing at least one of the groups selected from the hydrophilic group Pi means that R¹⁰ is a group selected from the hydrophilic group Pi or a group having at least one of the groups selected from the hydrophilic group Pi as a substituent. The same applies to a case where at least one of R⁸ or R⁹ is a group containing at least one of the groups selected from the hydrophilic group Pi.

In a case where the structure represented by General Formula (I) includes a structure in which X¹ to X³ are >CR²R³, and at least one R² in the structure in which X¹ to X³ are >CR²R³ is -NR⁸R⁹, -OR¹⁰, or a group selected from the above-mentioned hydrophilic group Pi, the structure represented by General Formula (I) preferably contains a group selected from the above-mentioned hydrophilic group Pi, and more preferably, the group selected from the hydrophilic group Pi is specifically the following group.

In one embodiment, it is preferable that at least one of the groups selected from the hydrophilic group Pi contained in the structure represented by General Formula (I) is a carboxy group, a sulfo group, or a phosphono group, and it is more preferable that the structure represented by General Formula (I) contains two or more groups selected from a carboxy group, a sulfo group, and a phosphono group.

In another embodiment, it is preferable that at least one of the groups selected from the hydrophilic group Pi contained in the structure represented by General Formula (I) is a betaine residue, a polyol residue, a sugar residue, a polyamino acid residue, or a polyalkyleneoxy group.

In one embodiment of this embodiment, it is preferable that at least one of the groups selected from the hydrophilic group Pi contained in the structure represented by General Formula (I) is a polyol residue, a sugar residue, a polyamino acid residue, or a polyalkyleneoxy group.

In this case, it is more preferable that the polyalkyleneoxy group further has, as a substituent, a group containing at least one group selected from a polyol residue, a sugar residue, and a polyamino acid residue.

In another embodiment of this embodiment, it is also preferable that at least one of the groups selected from the hydrophilic group Pi contained in the structure represented by General Formula (I) is a betaine residue.

### (Group selected from hydrophilic group Pi)

The compound according to the embodiment of the present invention does not have a phosphor moiety and has at least one group selected from the above-mentioned hydrophilic group Pi.

The type and number of groups selected from the above-mentioned hydrophilic group Pi contained in the compound according to the embodiment of the present invention are not particularly limited as long as the targeted drug conjugate can be imparted with sufficient hydrophilicity and aggregation of the conjugate can be suppressed.

For example, with regard to the type of the group selected from the hydrophilic group Pi contained in the compound according to the embodiment of the present invention, the compound preferably has at least one of an anionic group, a cationic group, a betaine residue, or a polyalkyleneoxy group, more preferably at least one of an anionic group, still more preferably at least one of a phosphono group, a phosphonooxy group, or a sulfo group, particularly preferably at least one of a phosphonooxy group or a sulfo group, and most preferably at least a sulfo group.

In addition, the number of groups selected from the above-mentioned hydrophilic group Pi contained in the compound according to the embodiment of the present invention is preferably 2 to 30, more preferably 2 to 20, still more preferably 2 to 16, and particularly preferably 2 to 12. In the above-mentioned description relating to each preferred range of the number of groups selected from the above-mentioned hydrophilic group Pi contained in the compound according to the embodiment of the present invention, the lower limit of the number of groups is preferably 3 or more, more preferably 4 or more, and still more preferably 5 or more.

n is an integer of 2 or more. In the compound according to the embodiment of the present invention, n is an integer of 2 or more, and therefore it is possible to impart excellent stability in the blood to the obtained targeted drug conjugate.

In the present invention, from the viewpoint of achieving more excellent stability in the blood, the lower limit value of n is preferably an integer of 3 or more, more preferably an integer of 6 or more, still more preferably an integer of 9 or more, and particularly preferably an integer of 12 or more. The upper limit value of n is not particularly limited and can be set to, for example, an integer of 72 or less, and is preferably an integer of 36 or less, more preferably an integer of 24 or less, and still more preferably an integer of 21 or less. That is, n is preferably an integer of 3 to 72, more preferably an integer of 6 to 36, still more preferably an integer of 9 to 24, and particularly preferably an integer of 12 to 21.

The above-mentioned preferred value of n can also be applied to n in the compound represented by General Formula (II) or (III) which will be described later. For the compound represented by General Formula (IV) which will be described later, the above-mentioned preferred value of n can be applied as a value of (v + 1) × m + 1, and for the compound represented by General Formula (V) which will be described later, the above-mentioned preferred value of n can be applied as a value of (((na + nb + nc) × r) + 1) × m + 1.

### <Compound represented by General Formula (II)>

Among the compounds according to the embodiment of the present invention, the compound in which the linking group that links the structural moieties M adjacent to each other is a group containing the structure represented by General Formula (I) is preferably represented by General Formula (II).

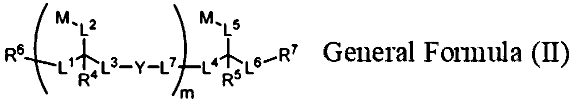

In the formula, R⁴ and R⁵ each represent a hydrogen atom, an alkyl group, an alkenyl group, an alkynyl group, an acyl group, an amino group, a hydroxy group, an alkoxy group, a sulfanyl group, an aryl group, or a heteroaryl group.

R⁶ and R⁷ each represent a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, at least one of R⁶ or R⁷ is a group containing a carboxy group or a group containing a substituent capable of binding to a target binding substance.

Q represents a group selected from the hydrophilic group Pi or a substituent capable of binding to the target binding substance.

L¹, L³, L⁴, L⁶, and L⁷ each represent a single bond or a linking group.

L² and L⁵ each represent a single bond or a physiologically cleavable linker.

In this regard, in a case where L² is a single bond, at least one of L¹ or L³ is a physiologically cleavable linker, and in a case where L⁵ is a single bond, at least one of L⁴ or L⁶ is a physiologically cleavable linker.

M represents the above-mentioned structural moiety M.

Y represents the above-mentioned structure represented by General Formula (I).

m is an integer of 1 or more.

R⁴ and R⁵ each represent a hydrogen atom, an alkyl group, an alkenyl group, an alkynyl group, an acyl group, an amino group, a hydroxy group, an alkoxy group, a sulfanyl group, an aryl group, or a heteroaryl group.

The preferred ranges of the alkyl group, alkenyl group, alkynyl group, acyl group, amino group, alkoxy group, aryl group, and heteroaryl group, which can be adopted as R⁴ or R⁵, are as described above.

The alkyl group, alkenyl group, alkynyl group, acyl group, amino group, alkoxy group, aryl group, and heteroaryl group, which can be adopted as R⁴ or R⁵, each may be unsubstituted or each may have a substituent, and examples of the substituent which may be contained in such groups include the substituents in the substituent group T which will be described later. For example, a halogen atom is preferable.

From the viewpoint of ease of synthesis, R⁴ and R⁵ are each preferably a hydrogen atom. In a case where an amino acid is used as a raw material, R⁴ and R⁵ are each often a hydrogen atom. In this regard, since the substituents in R⁴ and R⁵ do not significantly contribute to the fact that the targeted drug conjugate obtained from the compound according to the embodiment of the present invention retains a larger amount of a drug while suppressing aggregation and exhibits excellent stability in the blood, R⁴ and R⁵ may also be substituents other than hydrogen atoms (an alkyl group, an alkenyl group, an alkynyl group, an acyl group, an amino group, a hydroxy group, an alkoxy group, a sulfanyl group, an aryl group, or a heteroaryl group).

R⁶ and R⁷ each represent a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, at least one of R⁶ or R⁷ is a group containing a carboxy group or a group containing a substituent capable of binding to a target binding substance.

The preferred ranges of the alkyl group, alkenyl group, alkynyl group, aryl group, alkoxy group, amino group, acyl group, and heteroaryl group, which can be adopted as R⁶ or R⁷, are as described above.

The alkyl group, alkenyl group, alkynyl group, aryl group, alkoxy group, amino group, acyl group, and heteroaryl group, which can be adopted as R⁶ or R⁷, each may be unsubstituted or each may have a substituent.

Examples of the substituent which may be contained in the alkyl group, alkenyl group, alkynyl group, aryl group, alkoxy group, amino group, acyl group, and heteroaryl group in R⁶ or R⁷ include the substituents in the substituent group T which will be described later, among which an alkyl group, an acyl group, an alkoxy group, an amino group, Q, or a substituent obtained by combining two or more thereof is preferable, and an alkyl group, an acyl group, an alkoxy group, an amino group, -(O-L)_{g}R^{E}, -(L-O)_{g}R^{E}, a carboxy group, a substituent capable of binding to a target binding substance, or a substituent obtained by combining two or more of these substituents is more preferable.

Q which can be adopted as R⁶ or R⁷ represents a group selected from the hydrophilic group Pi or a substituent capable of binding to a target binding substance.

The description of the substituent capable of binding to a target binding substance which will be described later can be applied to the substituent capable of binding to a target binding substance.

Q is preferably a carboxy group or a substituent capable of binding to a target binding substance, and more preferably a substituent capable of binding to a target binding substance.

Preferred examples of R⁶ include the description of a substituent represented by -L⁸R^{6A} which will be described later. Specifically, the description of a substituent represented by -L⁸R^{6A} means a substituent obtained by a combination of a group which can be adopted as L⁸ and a group which can be adopted as R^{6A}.

R⁷ is preferably a group formed by substituting the following linking group with the following substituent. That is, the linking group is preferably one of an alkylene group, -O-, >C=O, and >NR^{A}, or a linking group obtained by combining two or more thereof, and more preferably an alkylene group, -O-, >C=O, >NR^{A}, -NR^{A}-alkylene, a group in which an -NR^{A}-alkylene group is bonded to a right side of a repeating unit (preferably having the repetition number of 1 to 20) represented by -[NR^{A}-alkylene-C(=O)]-, a group in which -NR^{A}-(L-O)_{g}-alkylene is bonded to a right side of a repeating unit (preferably having the repetition number of 1 to 20) represented by -[NR^{A}-alkylene-C(=O)]-, -NR^{A}-(L-O)_{g}-alkylene, or -C(=O)-(L-O)_{g}-alkylene. The substituent is preferably an alkyl group, a sulfanyl group, an acylamino group, a carbamoyl group, an aryl group, a heteroaryl group, or Q, and more preferably an alkyl group, an acylamino group, a carbamoyl group, or Q. R^{A} has the same definition as R^{A} which will be described later, and represents a hydrogen atom or a substituent. L and g have the same definitions as L and g described above.

R⁷ is also preferably a group containing a group selected from the hydrophilic group Pi. In this case, the alkylene group that can constitute R⁷ is preferably an alkylene group containing at least one of the groups selected from the hydrophilic group Pi, and more preferably an alkylene group having, as a substituent, at least one of the groups selected from the hydrophilic group Pi or an alkylene group substituted with an aryl group having, as a substituent, at least one of the groups selected from the hydrophilic group Pi.

L³, L⁴ and L⁷ each represent a single bond or a linking group, and are each preferably a single bond or one of an alkylene group, an alkenylene group, an alkynylene group, an arylene group, a heteroarylene group, -O-, -S-, >C=O, >NR^{A}, >S=O, >S(=O)₂, and >P(=O)OR^{B}, or a linking group obtained by combining two or more thereof. R^{A} and R^{B} have the same definitions as R^{A} and R^{B} which will be described later and represent a hydrogen atom or a substituent.

L¹ and L⁶ each represent a single bond or a linking group, and are each preferably a single bond, an alkylene group, an alkenylene group, an alkynylene group, an arylene group, a heteroarylene group, -O-, -S-, >C=O, >NR^{A}, >S=O, >S(=O)₂, or >P(=O)OR^{B}. R^{A} and R^{B} have the same definitions as R^{A} and R^{B} which will be described later and represent a hydrogen atom or a substituent.

The preferred ranges of the alkylene group, alkenylene group, alkynylene group, arylene group, and heteroarylene group, which can constitute L¹, L³, L⁴, L⁶, and L⁷, are as described above.

The alkylene group, alkenylene group, alkynylene group, arylene group, and heteroarylene group, which can constitute L¹, L³, L⁴, L⁶, and L⁷, each may be an unsubstituted group or a group having a substituent.

The substituent which may be contained in the alkylene group, alkenylene group, alkynylene group, arylene group, and heteroarylene group, which can constitute L¹, L³, L⁴, L⁶, and L⁷, is not particularly limited, and is preferably selected from the substituent group T which will be described later, and more preferably includes a halogen atom, an alkyl group, an acylamino group, a carbamoyl group, an aryl group, and a group selected from the hydrophilic group Pi. In addition, the substituent which may be contained in the alkylene group, alkenylene group, alkynylene group, arylene group, and heteroarylene group, which can constitute L¹, L³, L⁴, L⁶, and L⁷, may be further substituted with a substituent selected from the substituent group T which will be described later, and is preferably substituted with, for example, an amino group or a group selected from the hydrophilic group Pi.

In addition, the number of substituents which may be contained in the alkylene group, alkenylene group, alkynylene group, arylene group, and heteroarylene group, which can constitute L¹, L³, L⁴, L⁶, and L⁷, is not particularly limited as long as it can be adopted as a structure. The number of substituents can be set to at least one or more, and the upper limit value thereof is not particularly limited. For example, all of the hydrogen atoms in the alkylene group, alkenylene group, alkynylene group, arylene group, and heteroarylene group may be substituted with substituents.

The substituent which can be adopted as R^{A} in >NR^{A} and R^{B} in >P(=O)OR^{B}, which can constitute L¹, L³, L⁴, L⁶, and L⁷, is not particularly limited, and is preferably selected from the substituent group T which will be described later. R^{A} is preferably a hydrogen atom, an alkyl group, an alkenyl group, an alkynyl group, an aryl group, a heteroaryl group, or a group selected from the hydrophilic group Pi, more preferably a hydrogen atom or an alkyl group, and still more preferably a hydrogen atom. R^{B} is preferably a hydrogen atom, an alkyl group, an alkenyl group, an alkynyl group, an aryl group, or a heteroaryl group, more preferably a hydrogen atom or an alkyl group, and still more preferably a hydrogen atom.

It is noted that the alkyl group, alkenyl group, alkynyl group, aryl group, and heteroaryl group, which can be adopted as R^{A} and R^{B}, each may be an unsubstituted group or a group having a substituent.

In the linking group obtained by combining two or more of the alkylene group, alkenylene group, alkynylene group, arylene group, heteroarylene group, -O-, -S-, >C=O, >NR^{A}, >S=O, >S(=O)₂, and >P(=O)OR^{B}, which can constitute L³, L⁴, and L⁷, the types of the groups to be combined are not particularly limited as long as a reasonable chemical structure is obtained; but for example, 2 to 6 types are preferable, and 2 to 4 types are more preferable. It is noted that, in a case where each of an alkylene group, an alkenylene group, an alkynylene group, an arylene group, a heteroarylene group, -O-, -S-, >C=O, >NR^{A}, >S=O, >S(=O)₂, and >P(=O)OR^{B} is counted as one type, the maximum number of types is 12.

It is noted that, in the linking group obtained by combining two or more of the alkylene group, alkenylene group, alkynylene group, arylene group, heteroarylene group, -O-, -S-, >C=O, >NR^{A}, >S=O, >S(=O)₂, and >P(=O)OR^{B}, which can constitute L³, L⁴, and L⁷, the number of groups to be combined is not particularly limited and is preferably, for example, 2 to 10, more preferably 2 to 6, and still more preferably 2 to 4.
(i) L¹ and L⁶
   L¹ and L⁶ are each more preferably a single bond, >C=O, >NR^{A}, an arylene group, an alkylene group, -O-, or -S-, still more preferably a single bond, >C=O, >NR^{A}, an arylene group, or an alkylene group, and particularly preferably a single bond, >C=O, or >NR^{A}.
(ii) L³, L⁴, and L⁷
   L³, L⁴, and L⁷ are each more preferably a single bond, >C=O, >NR^{A}, an alkylene group, an alkenylene group, an alkynylene group, an arylene group, a heteroarylene group, or a combination of at least one of an alkylene group, an alkenylene group, an alkynylene group, an arylene group, a heteroarylene group, -O-, or -S- with >C=O and >NR^{A}, and still more preferably a single bond, >C=O, >NR^{A}, an alkylene group, an alkenylene group, an alkynylene group, an arylene group, a heteroarylene group, or a group that links -C(=O)NR^{A}- and >C=O, or >NR^{A}C(=O)- and >NR^{A} by at least one of an alkylene group, an alkenylene group, an alkynylene group, an arylene group, a heteroarylene group, -O-, or -S-.

L² and L⁵ each represent a single bond or a physiologically cleavable linker. In this regard, in a case where L² is a single bond, at least one of L¹ or L³ is a physiologically cleavable linker, and in a case where L⁵ is a single bond, at least one of L⁴ or L⁶ is a physiologically cleavable linker.

The physiologically cleavable linker which can be adopted as L² and L⁵ is as described above.

The physiologically cleavable linker may be introduced by synthesis using a conventional method, or a commercially available physiologically cleavable linker may be used.

m is an integer of 1 or more. The upper limit value of m is not particularly limited, and can be set to, for example, an integer of 30 or less and is preferably an integer of 20 or less, more preferably an integer of 10 or less, still more preferably an integer of 5 or less, and particularly preferably an integer of 3 or less. That is, m can be set to an integer of 1 to 30, and is preferably an integer of 1 to 20, more preferably an integer of 1 to 15, and still more preferably an integer of 1 to 10.

### <Compound represented by General Formula (III)>

The compound represented by General Formula (II) is preferably represented by General Formula (III).

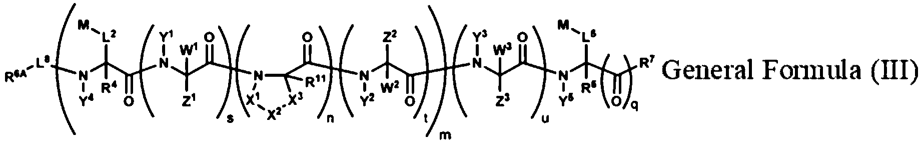

In the formula, Y¹ to Y⁵, Z¹ to Z³, and W¹ to W³ each represent a hydrogen atom, an alkyl group, an alkenyl group, an alkynyl group, an acyl group, an amino group, a hydroxy group, an alkoxy group, a sulfanyl group, an aryl group, a heteroaryl group, or a group selected from the above-mentioned hydrophilic group Pi.

R^{6A} represents a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, R^{6A} is a carboxy group or a substituent capable of binding to a target binding substance and/or R⁷ is a group containing a carboxy group or a group containing a substituent capable of binding to the target binding substance.

R⁴, R⁵, R⁷, R¹¹, L², L⁵, X¹ to X³, M, Q, m, and n have the same definitions as R⁴, R⁵, R⁷, R¹¹, L², L⁵, X¹ to X³, M, Q, m, and n described above.

L⁸ represents a single bond or a linking group.

s, t, and u each represent 0 or an integer of 1 or more.

q is an integer of 0 or 1.

Y¹ to Y⁵, Z¹ to Z³, and W¹ to W³ each represent a hydrogen atom, an alkyl group, an alkenyl group, an alkynyl group, an acyl group, an amino group, a hydroxy group, an alkoxy group, a sulfanyl group, an aryl group, a heteroaryl group, or a group selected from the above-mentioned hydrophilic group Pi.

The preferred ranges of the alkyl group, alkenyl group, alkynyl group, acyl group, amino group, alkoxy group, aryl group, and heteroaryl group, which can be adopted as Y¹ to Y⁵, Z¹ to Z³, or W¹ to W³, are as described above.

The alkyl group, alkenyl group, alkynyl group, acyl group, amino group, alkoxy group, aryl group, and heteroaryl group, which can be adopted as Y¹ to Y⁵, Z¹ to Z³, or W¹ to W³, each may be unsubstituted or each may have a substituent, and examples of the substituent which may be contained in such groups include the substituents in the substituent group T which will be described later. For example, an aryl group, a halogen atom, or a group selected from the above-mentioned hydrophilic group Pi is preferable.

In addition, the substituent which may be contained in the alkyl group, alkenyl group, alkynyl group, acyl group, amino group, alkoxy group, aryl group, and heteroaryl group, which can constitute Y¹ to Y⁵, Z¹ to Z³, or W¹ to W³, may be further substituted with a substituent selected from the substituent group T which will be described later, and is preferably substituted with, for example, a group selected from the above-mentioned hydrophilic group Pi.

Y¹ to Y³ are each preferably a hydrogen atom, an alkyl group, an aryl group, or a heteroaryl group, and more preferably a hydrogen atom or an alkyl group.

Y⁴ and Y⁵ are each preferably a hydrogen atom or an alkyl group and more preferably a hydrogen atom.

W¹ to W³ are each preferably a hydrogen atom, an alkyl group, an aryl group, or a heteroaryl group, and more preferably a hydrogen atom.

Z¹ to Z³ are each preferably an alkyl group, an aryl group, or a heteroaryl group, and more preferably an alkyl group.

In the compound represented by General Formula (III), in order to suppress aggregation of the targeted drug conjugate to be obtained, it is preferable that at least one of Z¹, Z², or Z³ is a group containing at least one of the groups selected from the hydrophilic group Pi, and it is more preferable that at least one of Z¹, Z², or Z³ is an alkyl group containing at least one of the groups selected from the hydrophilic group Pi.

The alkyl group containing at least one of the groups selected from the hydrophilic group Pi is preferably an alkyl group having, as a substituent, at least one of the groups selected from the hydrophilic group Pi, or an alkyl group substituted with an aryl group having, as a substituent, at least one of the groups selected from the hydrophilic group Pi.

Here, the phrase "at least one of Z¹, Z², or Z³ is a group containing at least one of the groups selected from the hydrophilic group Pi" means that at least one of the following condition (Z1), (Z2), or (Z3) is satisfied.

Condition (Z1): s is an integer of 1 or more, and Z¹ is a group containing at least one of the groups selected from the hydrophilic group Pi.

Condition (Z2): t is an integer of 1 or more, and Z² is a group containing at least one of the groups selected from the hydrophilic group Pi.

Condition (Z3): u is an integer of 1 or more, and Z³ is a group containing at least one of the groups selected from the hydrophilic group Pi.

In addition, the phrase "at least one of Z¹, Z², or Z³ is an alkyl group containing at least one of the groups selected from the hydrophilic group Pi" means that the phrase "a group containing at least one of the groups selected from the hydrophilic group Pi" in the conditions (Z1) to (Z3) is read as "an alkyl group containing at least one of the groups selected from the hydrophilic group Pi", and at least one of the condition (Z1), (Z2), or (Z3) is satisfied.

From the viewpoint of suppressing aggregation of the targeted drug conjugate to be obtained, each of the groups Z¹ to Z³ may contain two or more groups selected from the hydrophilic group Pi.

R^{6A} and L⁸ are determined so that R^{6A} is an unsubstituted group, and L⁸ is the longest group. In this regard, in a case where the group represented by -L⁸R^{6A} has Q, Q located at the most terminal side (R^{6A} side in -L⁸R^{6A}) is determined to be R^{6A}.

R^{6A} represents a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, R^{6A} is a carboxy group or a substituent capable of binding to a target binding substance and/or R⁷ is a group containing a carboxy group or a group containing a substituent capable of binding to the target binding substance.

The preferred ranges of the alkyl group, alkenyl group, alkynyl group, aryl group, alkoxy group, amino group, acyl group, and heteroaryl group, which can be adopted as R^{6A}, are as described above. In this regard, all of the groups are unsubstituted groups.

Q which can be adopted as R^{6A} has the same definition as Q described above, and the same applies to the preferred range thereof.

R^{6A} is preferably an alkyl group, a sulfanyl group, an aryl group, a heteroaryl group, or Q, more preferably an alkyl group or Q, and still more preferably an alkyl group, a carboxy group, or a substituent capable of binding to a target binding substance.

L⁸ represents a single bond or a linking group.

The linking group which can be adopted as L⁸ is preferably, for example, one of an alkylene group, an alkenylene group, an alkynylene group, an arylene group, a heteroarylene group, -O-, >C=O, and >NR^{A}, or a linking group obtained by combining two or more thereof. R^{A} represents a hydrogen atom or a substituent.

For the alkylene group, alkenylene group, alkynylene group, arylene group, heteroarylene group, and >NR^{A}, which can constitute L⁸, the description of the alkylene group, alkenylene group, alkynylene group, arylene group, heteroarylene group, and >NR^{A}, which can constitute L¹, L³, L⁴, L⁶, and L⁷ described above, can be preferably applied.

The substituent which may be contained in the alkylene group, alkenylene group, alkynylene group, arylene group, and heteroarylene group, which can constitute L⁸, is not particularly limited, and is preferably selected from the substituent group T which will be described later and is more preferably, for example, a halogen atom, an aryl group, or an alkyl group. In addition, the substituent which may be contained in the alkylene group, alkenylene group, alkynylene group, arylene group, and heteroarylene group, which can constitute L⁸, may be further substituted with a substituent selected from the substituent group T which will be described later, and is preferably substituted with, for example, a group selected from the above-mentioned hydrophilic group Pi.

In addition, the number of substituents which may be contained in the alkylene group, alkenylene group, alkynylene group, arylene group, and heteroarylene group, which can constitute L⁸, is not particularly limited as long as it can be adopted as a structure. The number of substituents can be set to at least one or more, and the upper limit value thereof is not particularly limited. For example, all of the hydrogen atoms in the alkylene group, alkenylene group, alkynylene group, arylene group, and heteroarylene group may be substituted with substituents.

In this regard, in a case where R^{6A} is a carboxy group or a substituent capable of binding to a target binding substance, L⁸ is preferably a linking group in which the number of shortest-distance atoms that connect >NY⁴ and R^{6A} is 3 or more.

With regard to the linking group L⁸, "the number of shortest-distance atoms that connect >NY⁴ and R^{6A}" means the number of atoms that constitute the shortest chain connecting >NY⁴ and R^{6A}. >NY⁴ means >NY⁴ directly bonded to L⁸.

L⁸ is more preferably a single bond or one of an alkylene group, -O-, >C=O, and >NR^{A}, or a linking group obtained by combining two or more thereof, and still more preferably a single bond, an alkylene group, -O-, >C=O, >NR^{A}, a group in which -NR^{A}-alkylene group is bonded to a right side of a repeating unit (preferably having the repetition number of 1 to 20) represented by -[NR^{A}-alkylene-C(=O)]-, -NR^{A}-(L-O)_{g}-alkylene, or -C(=O)-(L-O)_{g}- alkylene. L and g have the same definitions as L and g described above.

s, t, and u are each 0 or an integer of 1 or more.

The upper limit value of each of s, t, and u is not particularly limited, and can be set to, for example, 20 or less and is preferably 10 or less and more preferably 5 or less. That is, s, t, and u are each preferably an integer of 0 to 20, more preferably an integer of 0 to 10, and still more preferably an integer of 0 to 5. Above all, s, t, and u are each preferably an integer of 0 or 1.

It is noted that, in a case where a structure can be classified into both a structure parenthesized by t and a structure parenthesized by u, it is classified preferentially into a structure parenthesized by u.

q is an integer of 0 or 1 and is preferably 0.

<Compound represented by General Formula (IV)>

Among the compounds according to the embodiment of the present invention, a compound containing a structure in which the structural moiety M is bonded to any site of X¹, X², or X³ in the structure represented by General Formula (I) directly or through a linking group is preferably represented by General Formula (IV).

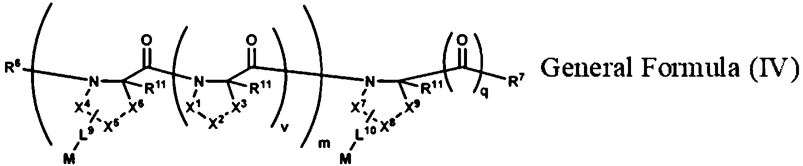

In the formula, X⁴ to X⁹ each represent -O-, -S-, >NR¹, or >CR²R³.

In this regard, at least one of X⁴, X⁵, or X⁶ is >N-L⁹-M or >C(R²)-L⁹-M, and at least one of X⁷, X⁸, or X⁹ is >N-L¹⁰-M or >C(R²)-L¹⁰-M.

R¹ to R³ which are neither -L⁹-M nor -L¹⁰-M have the same definitions as R¹ to R³ in General Formula (I) described above.

L⁹ and L¹⁰ each represent a physiologically cleavable linker.

R⁶ and R⁷ each represent a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, at least one of R⁶ or R⁷ is a group containing a carboxy group or a group containing a substituent capable of binding to a target binding substance.

Q represents a group selected from the above-mentioned hydrophilic group Pi or a substituent capable of binding to a target binding substance.

m is an integer of 1 or more, q is an integer of 0 or 1, and v is 0 or an integer of 1 or more.

R¹¹, X¹ to X³, and M have the same definitions as R¹¹, X¹ to X³, and M described above.

Unless otherwise specified, the descriptions of X¹ to X³ in General Formula (I) described above can be applied to X⁴ to X⁹, except that at least one of X⁴, X⁵, or X⁶ is >N-L⁹-M or >C(R²)-L⁹-M and at least one of X⁷, X⁸, or X⁹ is >N-L¹⁰-M or >C(R²)-L¹⁰-M.

That is, the description of X¹ described above can be applied to X⁴ and X⁷, the description of X² described above can be applied to X⁵ and X⁸, and the description of X³ described above can be applied to X⁶ and X⁹, and the descriptions of R¹, R², and R³ in General Formula (I) described above can be respectively applied to R¹, R², and R³ which are neither -L⁹-M nor -L¹⁰-M.

In >NR¹ and >CR²R³ which do not have either -L⁹-M or -L¹⁰-M among X⁴ to X⁹, R¹ is preferably an alkyl group, and R² and R³ are each preferably a hydrogen atom.

Regarding the two groups among X⁴ to X⁶, which do not have -L⁹-M described above, it is preferable that at least one thereof is >CR²R³, it is more preferable that at least one thereof is >CR²R³ and the remaining one is -O-, -S-, or >CR²R³, and it is still more preferable that both the two are >CR²R³.

Regarding the two groups among X⁷ to X⁹, which do not have -L¹⁰-M described above, it is preferable that at least one thereof is >CR²R³, it is more preferable that at least one thereof is >CR²R³ and the remaining one is -O-, -S-, or >CR²R³, and it is still more preferable that both the two are >CR²R³.

Among X⁴ to X⁶, >N-L⁹-M or >C(R²)-L⁹-M is preferably >C(R²)-L⁹-M, and more preferably >CH-L⁹-M.

Among X⁷ to X⁹, >N-L¹⁰-M or >C(R²)-L¹⁰-M is preferably >C(R²)-L¹⁰-M, and more preferably >CH-L¹⁰-M.

Among X⁴ to X⁶, the group having -L⁹-M is not particularly limited and is preferably X⁵.

Among X⁷ to X⁹, the group having -L¹⁰-M is not particularly limited and is preferably X⁸.

L⁹ and L¹⁰ each represent a physiologically cleavable linker.

For the physiologically cleavable linker as L⁹ and L¹⁰, the description of the physiologically cleavable linker described above can be applied.

R⁶ and R⁷ each represent a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, at least one of R⁶ or R⁷ is a group containing a carboxy group or a group containing a substituent capable of binding to a target binding substance.

The description of R⁶ and R⁷ in General Formula (II) described above can be applied to R⁶ and R⁷. In addition, the description of Q in General Formula (II) described above can be applied to Q which can be adopted as R⁶ or R⁷.

m is an integer of 1 or more, and the description of m in General Formula (II) described above can be applied thereto.

q is an integer of 0 or 1 and is preferably 0.

v is 0 or an integer of 1 or more, preferably an integer of 1 to 36, more preferably an integer of 2 to 24, still more preferably an integer of 3 to 36, particularly preferably an integer of 4 to 24, and above all, preferably an integer of 5 to 18.

### <Compound represented by General Formula (V)>

The compound represented by General Formula (IV) is preferably represented by General Formula (V).

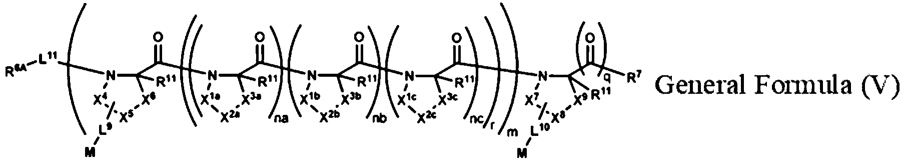

In the formula, X^{1a}, X^{1b}, X^{1c}, X^{2a}, X^{2b}, x^{2c}, X^{3a}, X^{3b}, and X^{3c} each represent -O-, -S-, >NR¹, or >CR²R³.

L¹¹ represents a single bond or a linking group.

R^{6A} represents a hydrogen atom, a hydroxy group, a sulfanyl group, an alkyl group, an alkenyl group, an alkynyl group, an aryl group, an alkoxy group, an amino group, an acyl group, a heteroaryl group, or Q. In this regard, R^{6A} is a carboxy group or a substituent capable of binding to a target binding substance and/or R⁷ is a group containing a carboxy group or a group containing a substituent capable of binding to the target binding substance.

R¹ to R³, R⁷, R¹¹, X⁴ to X⁹, L⁹, L¹⁰, M, Q, m, and q have the same definitions as R¹ to R³, R⁷, R¹¹, X⁴ to X⁹, L⁹, L¹⁰, M, Q, m, and q described above.

na, nb, and nc are each 0 or an integer of 1 or more. r is an integer of 1 or more.

The description of X¹ in General Formula (I) described above can be applied to X^{1a}, X¹⁶, and X^{1c}.

The description of X² in General Formula (I) described above can be applied to X^{2a}, X^{2b}, and X^{2c}.

The description of X³ in General Formula (I) described above can be applied to X^{3a}, X^{3b}, and X^{3c}.

X^{1a}, X^{1b}, and X^{1c}, and X^{3a}, X^{3b}, and X^{3c} are each preferably >NH or >CH₂, and more preferably >CH₂.

It is preferable that at least one of X^{2a}, X^{2b}, or X^{2c} is a group containing a group selected from the above-mentioned hydrophilic group Pi; it is more preferable that X^{2b} is a group containing a group selected from the above-mentioned hydrophilic group Pi, and X^{2a} and X^{2c} are each >NH or >CH₂; and it is still more preferable that X^{2b} is a group containing a group selected from the above-mentioned hydrophilic group Pi, and X^{2a} and X^{2c} are each >CH₂.

The group containing a group selected from the above-mentioned hydrophilic group Pi, which can be adopted as X^{2a}, X^{2b}, and X^{2c}, is preferably >NR¹, >CR²H, or a group selected from the above-mentioned hydrophilic group Pi, in which R¹ and R² are each an alkyl group, an arylacylamino group, or an arylaminocarbonyl group each containing one or more groups selected from the above-mentioned hydrophilic group Pi; more preferably a group represented by >NR¹ or >CR²H, in which R¹ and R³ are each an arylacylamino group or an arylaminocarbonyl group each containing one or more groups selected from the above-mentioned hydrophilic group Pi; still more preferably a group represented by >NR¹ or >CR²H, in which R¹ and R³ are each an arylacylamino group or an arylaminocarbonyl group each containing two or more groups selected from the above-mentioned hydrophilic group Pi; and particularly preferably a group represented by >NR¹ or >CR²H, in which R¹ and R³ are each an arylacylamino group or an arylaminocarbonyl group each having two or more alkoxy groups each containing a group selected from the above-mentioned hydrophilic group Pi.

R^{6A} and L¹¹ are determined so that R^{6A} is an unsubstituted group, and L¹¹ is the longest group. In this regard, in a case where the group represented by -L¹¹R^{6A} has Q, Q located at the most terminal side (R^{6A} side in -L¹¹R^{6A}) is determined to be R^{6A}.

The description of R^{6A} in General Formula (III) described above can be applied to R^{6A}.

L¹¹ represents a single bond or a linking group.

The description of the linking group which can be adopted as L⁸ in General Formula (III) described above can be applied to the linking group that can be adopted as L¹¹.

L¹¹ is more preferably a single bond or one of an alkylene group, -O-, >C=O, and >NR^{A}, or a linking group obtained by combining two or more thereof, still more preferably a single bond, an alkylene group, >NR^{A}, >C=O, or a linking group obtained by combining an alkylene group with >C=O or >NR^{A}, and particularly preferably a single bond, an alkylene group, >NR^{A}, or >C=O.

na, nb, and nc are each 0 or an integer of 1 or more. r is an integer of 1 or more.

na, nb, and nc are each preferably an integer of 1 to 10, more preferably an integer of 1 to 5, and still more preferably an integer of 1 to 3.

r is preferably an integer of 1 to 5.

In a case where the compound represented by any one of General Formula (II), General Formula (III), General Formula (IV), or General Formula (V) among the compounds according to the embodiment of the present invention is obtained by using a peptide synthesis method, the structure is, in general, such that the C-terminal structure is on the right side of the paper surface, and the N-terminal structure is on the left side of the paper surface.

The compound according to the embodiment of the present invention preferably contains at least one of a carboxy group or a substituent capable of binding to a target binding substance in the substituent represented by Q.

The compound according to the embodiment of the present invention can be bound to a target binding substance through a carboxy group or a substituent capable of binding to the target binding substance which will be described later, and therefore a desired targeted drug conjugate can be obtained. The carboxy group can be easily derivatized by a conventional method to give a substituent capable of binding to a target binding substance.

In the present invention, for convenience, the substituent capable of binding to a target binding substance does not include a carboxy group, and the "substituent capable of binding to a target binding substance" includes a substituent capable of binding to a target binding substance, which is derived from a carboxy group. In this regard, as described above, it is also possible to be bound to the target binding substance through a carboxy group.

In the compound according to the embodiment of the present invention, the position at which the carboxy group or the substituent capable of binding to a target binding substance is present is not particularly limited, but the carboxy group or the substituent capable of binding to a target binding substance is preferably present in a structure other than the structure represented by General Formula (I) and the structural moiety M, and in the compound represented by General Formula (II) or (IV), the carboxy group or the substituent capable of binding to a target binding substance is present in at least one of R⁶ or R⁷, and in the compound represented by General Formula (III) or (V), the carboxy group or the substituent capable of binding to a target binding substance is present in at least one of R^{6A} or R⁷.

The number of the carboxy groups or substituents capable of binding to a target binding substance in the compound according to the embodiment of the present invention may be at least one or more in total, and is preferably 1 to 3, more preferably 1 or 2, and still more preferably 1 from the viewpoint of quantifying the substance to be detected.

In addition, the compound according to the embodiment of the present invention is a compound having at least one group selected from the above-mentioned hydrophilic group Pi. From the viewpoint of imparting sufficient hydrophilicity to the obtained targeted drug conjugate and suppressing aggregation, the compound according to the embodiment of the present invention preferably has 1 to 20 groups selected from the above-mentioned hydrophilic group Pi, more preferably 1 to 16 groups, and still more preferably 1 to 12 groups in total at positions other than the structural moiety M.

The position of the group selected from the above-mentioned hydrophilic group Pi is not particularly limited unless otherwise specified, and preferred examples thereof include X¹ to X³ or R¹¹ in General Formula (I), (III), or (IV), X¹ to X³ or R¹¹ in Y of General Formula (II), and X^{1a}, X^{1b}, X^{1c}, X^{2a}, X^{2b}, X^{2c}, X^{3a}, X^{3b}, X^{3c}, or R¹¹ in General Formula (V).

In addition, the compound according to the embodiment of the present invention is a compound that does not have a phosphor moiety (a structural moiety consisting of an organic compound that exhibits fluorescence). That is, the compound according to the embodiment of the present invention is a compound that does not have a phosphor moiety which can be adopted as M described in paragraph [0045] of WO2023/032995A.

Specific examples of the compound according to the embodiment of the present invention will be shown below, but the present invention is not limited to these compounds.

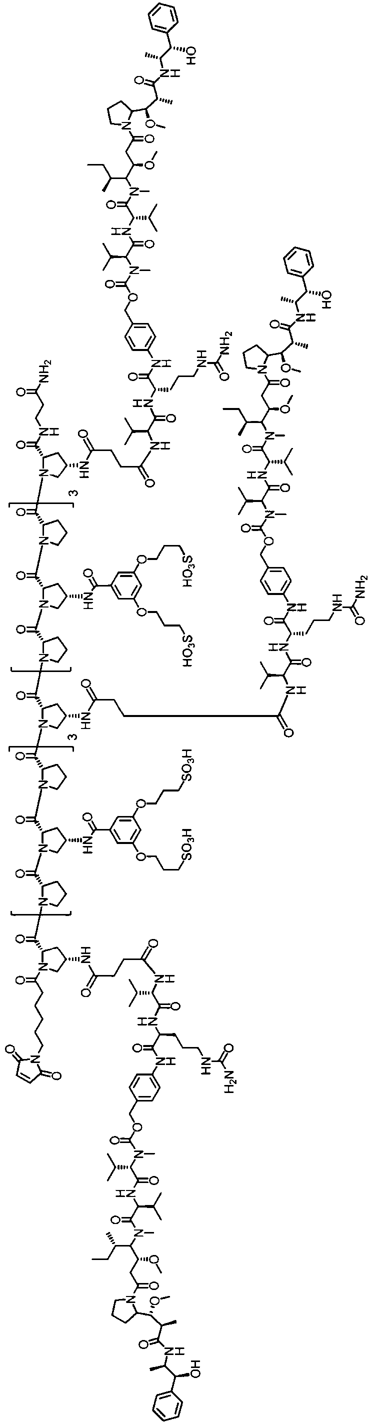

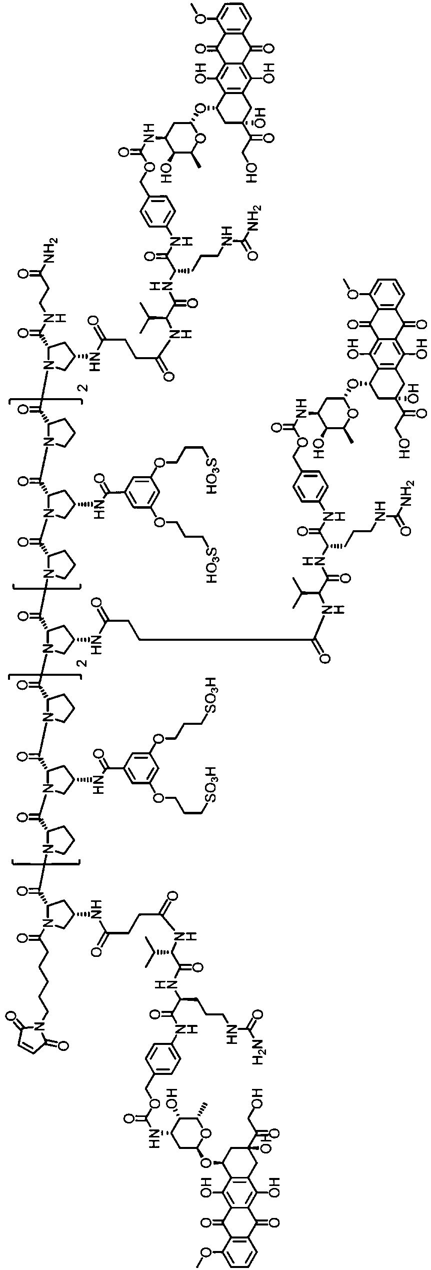

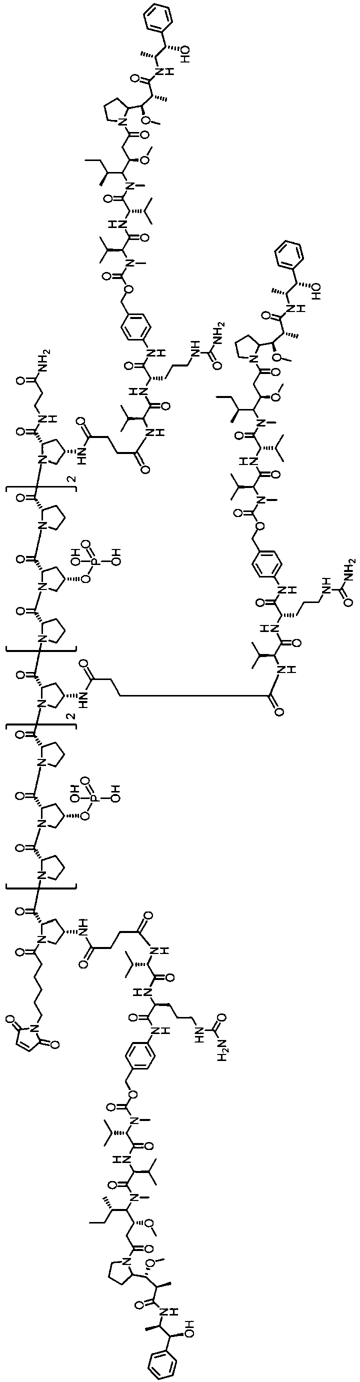

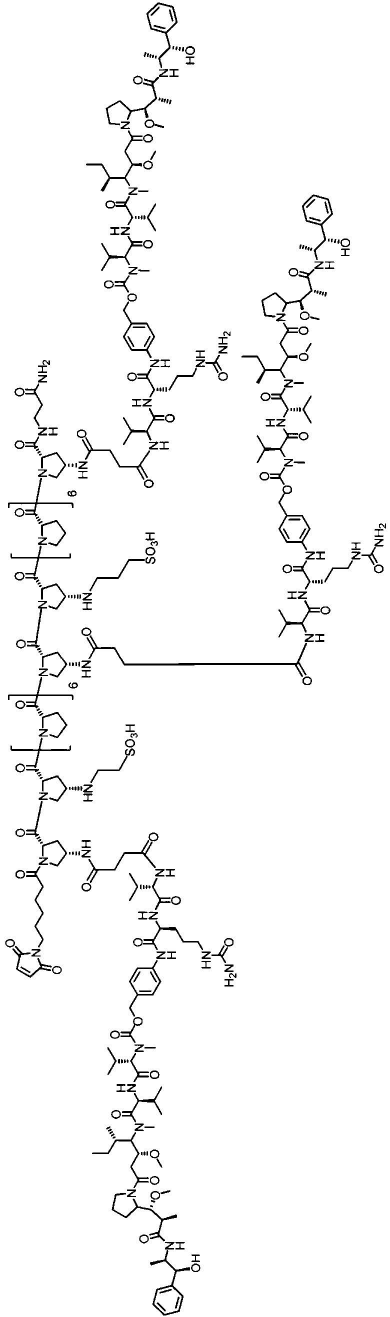

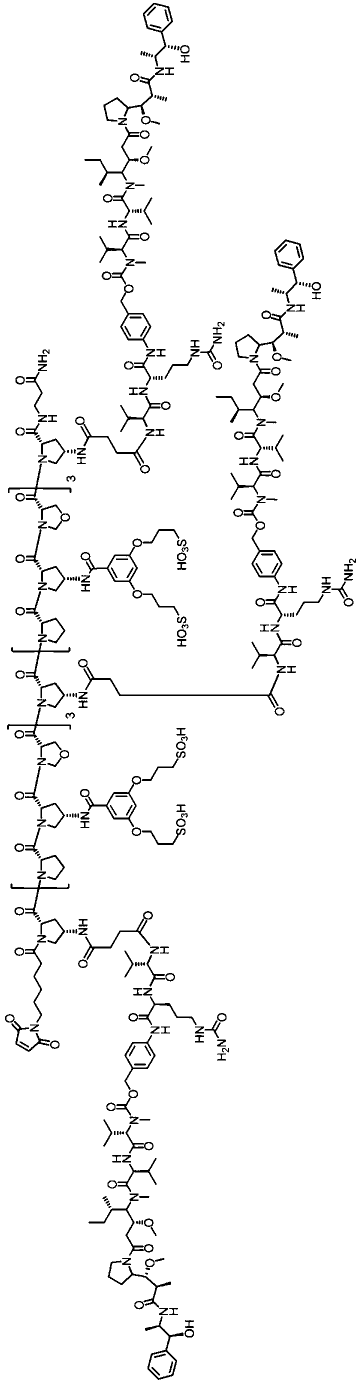

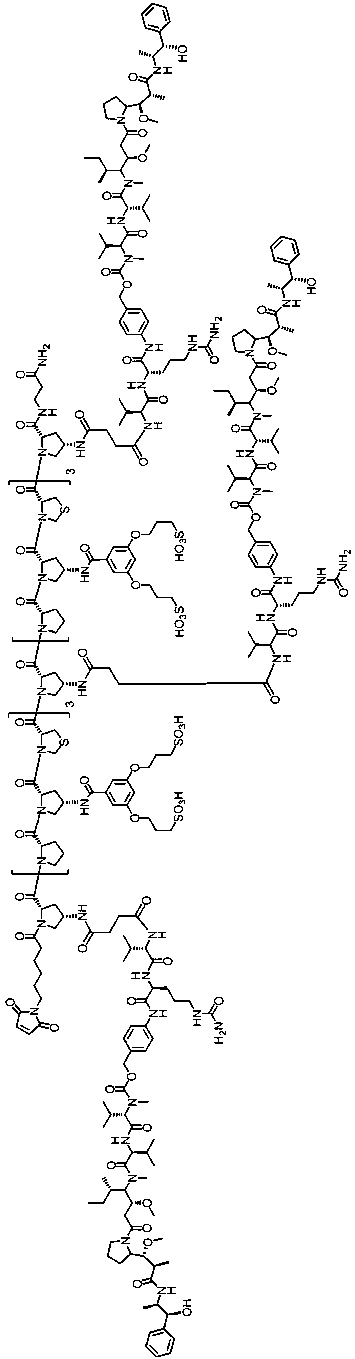

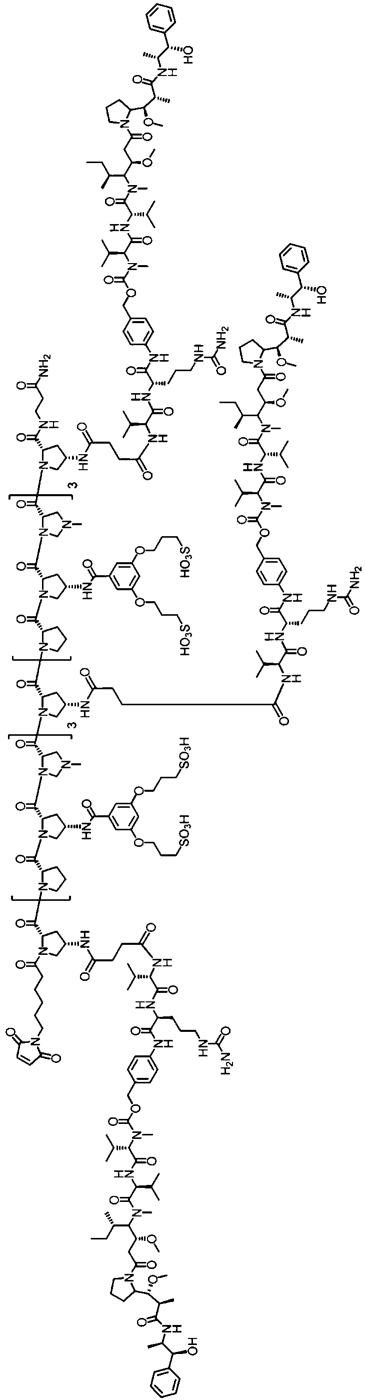

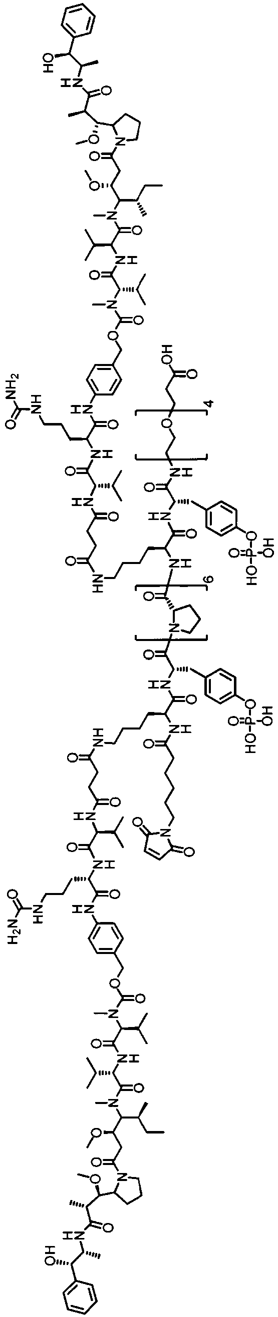

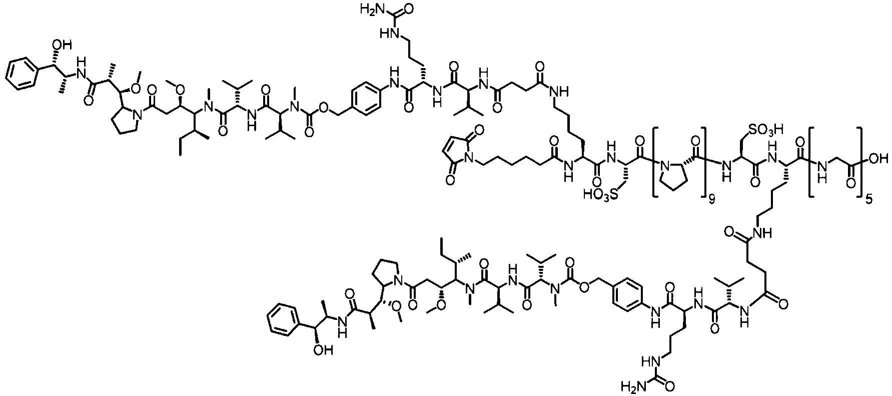

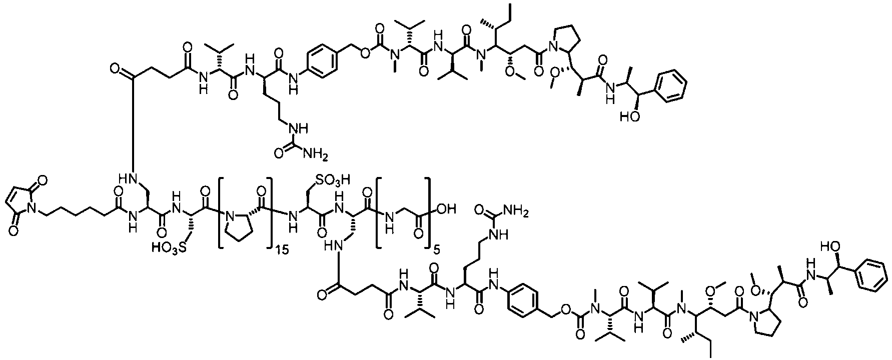

The compound according to the embodiment of the present invention can be bound to target binding substances such as a protein (including a peptide), an amino acid, a nucleic acid, a nucleotide, an enzyme, a sugar chain, a lipid, an antibody, an antibody fragment, a cell surface receptor antagonist, and a cell surface receptor agonist through at least one substituent capable of binding to a target binding substance contained in the compound, and the resulting structure can be used as a targeted drug conjugate.

The substituent capable of binding to a target binding substance can be used without any particular limitation as long as it is a group that acts on (including attaches to) or binds to the target binding substance, and examples thereof include the substituents described in WO2002/026891A. Specifically, the description of the electrophilic group and the nucleophilic group described in Table 2 of WO2002/026891A and the reactive group Rx described on page 18, line 16 to page 19, line 13 of WO2002/026891A can be applied to the present invention.

Specific examples of the "substituent capable of binding to a target binding substance" include the following structures. In the following structures, X represents a halogen atom such as an iodine atom or a bromine atom. * represents a bonding site.

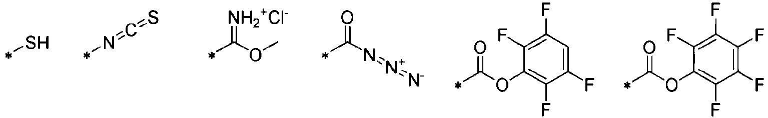

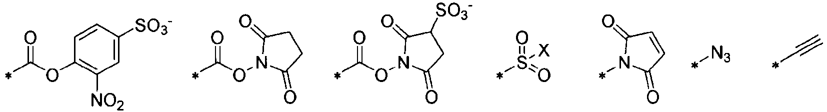

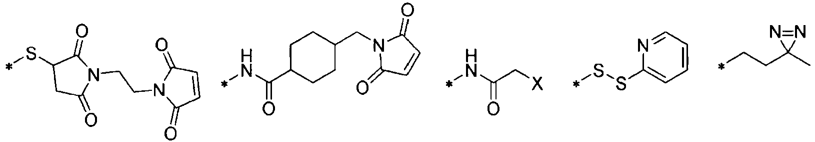

In addition to the above, a peptide structure (a polyamino acid structure), a long-chain alkyl group, or the like can also be used as the "substituent capable of binding to a target binding substance".

Above all, preferred examples of the peptide structure, long-chain alkyl group, or the like include an N-hydroxysuccinimide ester structure (an NHS ester structure), a succinimide structure, a maleimide structure, an azide group, an acetylene group, a peptide structure (a polyamino acid structure), a long-chain alkyl group (preferably having 12 to 30 carbon atoms), and a quaternary ammonium group.

Among the compounds according to the embodiment of the present invention, specific examples of the compound having at least one substituent capable of binding to a target binding substance also include a form in which the carboxy group in the above-mentioned exemplary compound according to the embodiment of the present invention is appropriately substituted with the substituent capable of binding to a target binding substance. The present invention is not limited to these compounds.

The compound according to the embodiment of the present invention can be synthesized by a conventional method. For example, the compound according to the embodiment of the present invention can be synthesized based on peptide synthesis such as solid phase peptide synthesis, and a method that uses an automated peptide synthesizer described in WO2018/174078A can also be preferably applied. The physiologically active substance moiety and the radioisotope-containing moiety can also be synthesized based on a conventional method and introduced into the compound according to the embodiment of the present invention. For example, reference can be made to the description in WO2015/115091A relating to the synthesis of an antibody drug conjugate.

The compound having a substituent capable of binding to a target binding substance can also be synthesized by a conventional method. For example, reference can be made to Bioconjugate Techniques (Third Edition, written by Greg T. Hermanson).

### <Targeted drug conjugate>

The targeted drug conjugate according to the embodiment of the present invention is a substance in which the compound according to the embodiment of the present invention is bound to a target binding substance. The compound according to the embodiment of the present invention makes it possible to obtain a targeted drug conjugate that retains a larger amount of a drug while suppressing aggregation and has excellent stability in the blood. The binding between the compound according to the embodiment of the present invention and the target binding substance may be in a form in which the compound according to the embodiment of the present invention is directly bound to the target binding substance or in a form in which the compound according to the embodiment of the present invention is linked to the target binding substance through a linking group.

The targeted drug conjugate according to the embodiment of the present invention is a substance in which the compound according to the embodiment of the present invention is bound to a target binding substance, and is preferably an antibody drug conjugate (ADC) in which the compound according to the embodiment of the present invention is bound to an antibody which is the target binding substance.

The term "target binding substance" refers to a substance that has the property of selectively binding to or associating with a specific target substance such as a tumor cell antigen. In addition, the phrase "selectively" binding to or associating with means that the target binding substance preferentially associates with or binds to a desired target over other targets. In a case where the target binding substance has these properties, the targeted drug conjugate according to the embodiment of the present invention can deliver the drug obtained from the above-mentioned structural moiety M to a target cell or inside the target cell.

The number of the above-mentioned structural moieties M in one molecule of the targeted drug conjugate according to the embodiment of the present invention is at least 2 or more, as one or more molecules of the compound according to the embodiment of the present invention are bound to one molecule of the target binding substance.

In the targeted drug conjugate according to the embodiment of the present invention, the number of the above-mentioned structural moieties M linked to one molecule of the target binding substance can be increased by binding two or more molecules of the compound according to the embodiment of the present invention to one molecule of the target binding substance. In this case, the number of the above-mentioned structural moieties M in one molecule of the targeted drug conjugate according to the embodiment of the present invention (corresponding to the number of the above-mentioned structural moieties M with respect to one molecule of the target binding substance in the targeted drug conjugate according to the embodiment of the present invention) is 4 or more, preferably 6 or more, and more preferably 8 or more. That is, the number of the above-mentioned structural moieties M in one molecule of the targeted drug conjugate according to the embodiment of the present invention is 4 to 20, preferably 6 to 20, more preferably 8 to 20, still more preferably 10 to 20, and particularly preferably 12 to 20.

In addition, the number of groups selected from the above-mentioned hydrophilic group Pi in one molecule of the targeted drug conjugate according to the embodiment of the present invention is preferably 2 to 30, more preferably 6 to 30, and still more preferably 8 to 20.

Preferred examples of the target binding substance include a protein (including a peptide), an amino acid, a nucleic acid, a nucleotide, an enzyme, a sugar chain, a lipid, an antibody, an antibody fragment, an antigen, a cell surface receptor antagonist, and a cell surface receptor agonist. Preferred examples of the lipid include a phospholipid, a fatty acid, and a sterol, among which a phospholipid is more preferable. Among these target binding substances, an antibody is preferable as the target binding substance.

More specifically, examples of the target binding substance include the descriptions relating to target-directed moieties such as an antibody, an antibody fragment, a cell surface receptor antagonist, a cell surface receptor agonist, and a protein, which are described in paragraphs [0079] to [0084] of JP2022-526651A.

In the targeted drug conjugate according to the embodiment of the present invention, an anticancer agent, an anti-infective drug, a neuroprotective drug, an antihyperlipidemic drug, a nucleic acid medicine, or the like can be used as the drug, and examples of a target to which the target binding substance in the targeted drug conjugate according to the embodiment of the present invention using these drugs binds include a tumor, an infected cell, a nerve cell, an immune cell, and a lipid.

Examples of the substance targeted by the targeted drug conjugate according to the embodiment of the present invention include a cell surface receptor such as a tumor cell antigen, more specific examples of which include the description relating to the antigen described in paragraph [0104] of JP2022-526651A.

Specific forms of the binding between the compound according to the embodiment of the present invention and the target binding substance through interaction include, for example, the forms described below.

For example, mention may be made of:
i) a non-covalent bond (for example, a hydrogen bond or an ionic bond including chelate formation) or a covalent bond between a peptide in the compound according to the embodiment of the present invention and a peptide in the target binding substance,
ii) a Van der Waals force between a long-chain alkyl group in the compound according to the embodiment of the present invention and a lipid bilayer, a lipid, or the like in the target binding substance,
iii) an amide bond formed by a reaction between an N-hydroxysuccinimide ester (an NHS ester) in the compound according to the embodiment of the present invention and an amino group in the target binding substance,
iv) a thioether bond formed by a reaction between a maleimide structure in the compound according to the embodiment of the present invention and a sulfanyl group (-SH) in the target binding substance, and
v) a formation of a triazole ring by a Click reaction between an azido group in the compound according to the embodiment of the present invention and an acetylene group in the target binding substance, or by a Click reaction between an acetylene group in the compound according to the embodiment of the present invention and an azido group in the target binding substance.

In this regard, in the form of i) described above, the peptide in the compound according to the embodiment of the present invention is not particularly limited as long as it is a peptide that is capable of forming a non-covalent bond or covalent bond with the peptide in the target binding substance, and preferred examples of the position where such a peptide is provided include R⁶ or R⁷ in General Formula (II) or (IV).

In addition to the forms i) to v) described above, binding can also be carried out in the form described in, for example, Lucas C. D. de Rezende and Flavio da Silva Emery. A Review of the Synthetic Strategies for the Development of BODIPY Dyes for Conjugation with Proteins, Orbital: The Electronic Journal of Chemistry, 2013, Vol 5, No. 1, pp. 62-83. In addition, the method described in the same document can also be appropriately referred to for the production of the targeted drug conjugate according to the embodiment of the present invention.

### <Medicine including targeted drug conjugate>

In the medicine including the targeted drug conjugate according to the embodiment of the present invention, the targeted drug conjugate according to the embodiment of the present invention is not particularly limited in the form of the targeted drug conjugate, including, for example, a solution form in which the targeted drug conjugate is dissolved in an aqueous medium such as saline or a phosphate buffer solution, and a solid form such as a fine particle powder or a freeze-dried powder, and the form of the targeted drug conjugate can be appropriately selected depending on the purpose of use and the like.

For example, in a case where the targeted drug conjugate according to the embodiment of the present invention is used as an ADC medicinal product, the targeted drug conjugate can also be used as a medicine including the targeted drug conjugate in any of the forms described above.

### [[Medicinal use]]

The targeted drug conjugate according to the embodiment of the present invention can be applied to medicinal use. In this case, the present invention further relates to a medicine including the above-mentioned targeted drug conjugate, and the same applies to a medicine suitable as the targeted drug conjugate. The medicine may include other additives.

The present invention further provides:
(A) use of the targeted drug conjugate for manufacturing a medicine;
(B) the targeted drug conjugate for use in the treatment of a subject;
(C) a treatment method including administering an effective dose of the targeted drug conjugate to a subject; and
(D) a method of administering a medicine to a subject, in which the medicine includes the targeted drug conjugate.

According to the above-mentioned treatment method, by using the targeted drug conjugate or the medicine according to the embodiment of the present invention, it is possible to treat a subject while retaining a larger amount of a drug and suppressing aggregation, and exhibiting excellent stability in the blood.

Here, the term "subject" refers to a mammal such as a human, a mouse, a monkey, or a domestic animal in need of prevention or therapy, and preferably a human in need of prevention or therapy.

The term "treatment" refers to prevention, therapy, or the like of a variety of diseases.

The term "prevention" refers to inhibition of the onset of a disease, reduction of the risk of the onset of a disease, delay of the onset of a disease, or the like.

The term "therapy" refers to improvement of, suppression (maintenance or delay) of the progression of, or the like of a target disease or condition.

The "targeted drug conjugate" and the "targeted drug conjugate included in a medicine" are preferably a targeted drug conjugate in which two or more molecules of the compound according to the embodiment of the present invention are bound to one molecule of the target binding substance, and more preferably an antibody drug conjugate (ADC) in which the "target binding substance" is an antibody. The above descriptions relating to the target binding substance, antibody, targeted drug conjugate, and antibody drug conjugate (ADC) can be applied to the target binding substance, antibody, targeted drug conjugate, and antibody drug conjugate (ADC), respectively.

The above-mentioned "disease" means a disease associated with a substance targeted by the above-mentioned targeted drug conjugate according to the embodiment of the present invention, and the medicine according to the embodiment of the present invention is used for therapy of this disease.

Examples of the disease for which the medicine according to the embodiment of the present invention is used include a cancer, a tumor, an autoimmune disease, an infectious disease, and a neurodegenerative disease. More specifically, examples of the cancer include the description relating to the cancers described in paragraphs [0176] to [0179] of JP2022-526651A.

The above-mentioned medicine can be prepared according to a conventional method without any particular limitations.

In the methods (C) and (D) described above, conditions of a commonly used method or the like can be appropriately selected except for the use of the targeted drug conjugate or the medicine according to the embodiment of the present invention.

### - Substituent group T -

In the present invention, in a case where the substituent is simply described as a substituent, the description of the corresponding substituent in the substituent group T can be referred to and applied in addition to the description of each substituent described at the beginning of the present specification. For example, in a case where only an "alkyl group" is simply described, the description of the "alkyl group" in the substituent group T can also be referred to and applied in addition to the description of the alkyl group defined at the beginning of the present specification. The same applies to other substituents other than the "alkyl group".

In addition, in the present invention, examples of the substituent which may be contained in a certain substituent such as an "alkyl group" include a substituent selected from the following substituent group T. In addition, in a case where a certain substituent such as an "alkyl group" has a substituent and further has a substituent, examples of the substituent which is contained in the certain substituent such as an "alkyl group" include a substituent obtained by combining two or more substituents selected from the following substituent group T.

Furthermore, in the present invention, in a case where an alkyl group is described to be distinguished from a cyclic (cyclo)alkyl group, the alkyl group is used in the sense of including a linear alkyl group and a branched alkyl group. On the other hand, in a case where an alkyl group is not described to be distinguished from a cyclic alkyl group, and unless otherwise specified, the alkyl group is used in the sense of including a linear alkyl group, a branched alkyl group, and a cycloalkyl group. The same applies to groups (an alkoxy group, an alkylthio group, an alkenyloxy group, and the like) containing a group capable of adopting a cyclic structure (an alkyl group, an alkenyl group, an alkynyl group, or the like) and compounds containing a group capable of adopting a cyclic structure. In a case where the group can form a cyclic skeleton, the lower limit of the number of atoms in the group forming the cyclic skeleton is 3 or more, preferably 4 or more, and more preferably 5 or more, regardless of the lower limit of the number of atoms specifically described below for the group that can adopt this structure.

In the description of the substituent group T below, a group having a linear or branched structure and a group having a cyclic structure are sometimes described separately, for example, an alkyl group and a cycloalkyl group, in order to clearly distinguish between the group having a linear or branched structure and the group having a cyclic structure.

The groups included in the substituent group T include the following groups:
an alkyl group (preferably having 1 to 20 carbon atoms, more preferably having 1 to 12 carbon atoms, still more preferably having 1 to 8 carbon atoms, even still more preferably having 1 to 6 carbon atoms, and particularly preferably having 1 to 3 carbon atoms), an alkenyl group (preferably having 2 to 20 carbon atoms, more preferably having 2 to 12 carbon atoms, still more preferably having 2 to 6 carbon atoms, and even still more preferably having 2 to 4 carbon atoms), an alkynyl group (preferably having 2 to 20 carbon atoms, more preferably having 2 to 12 carbon atoms, still more preferably having 2 to 6 carbon atoms, and even still more preferably having 2 to 4 carbon atoms), a cycloalkyl group (preferably having 3 to 20 carbon atoms), a cycloalkenyl group (preferably having 4 to 20 carbon atoms), an aryl group (which may be a monocyclic group or may be a fused ring group (preferably a fused ring group having 2 to 6 rings). In a case of a fused ring group, the fused ring group is preferably a 6- or 8-membered ring, and more preferably a 6-membered ring. The monocyclic ring is preferably a 6-membered ring. The aryl group preferably has 6 to 40 carbon atoms, more preferably 6 to 30 carbon atoms, still more preferably 6 to 26 carbon atoms, and particularly preferably 6 to 10 carbon atoms), a heterocyclic group (which may be a group having at least one nitrogen atom, oxygen atom, sulfur atom, phosphorus atom, silicon atom, or selenium atom as a ring-constituting atom, and may be a monocyclic group or a fused ring group (preferably a fused ring group having 2 to 6 rings). The number of ring members in the ring in the monocyclic group and the ring constituting the fused ring group is preferably 5 to 7, and more preferably 5 or 6. The number of carbon atoms in the heterocyclic group is preferably 2 to 40, and more preferably 2 to 20. The heterocyclic group includes an aromatic heterocyclic group (a heteroaryl group) and an aliphatic heterocyclic group (an aliphatic heterocyclic group.), an alkoxy group (preferably having 1 to 20 carbon atoms, more preferably having 1 to 12 carbon atoms, still more preferably having 1 to 8 carbon atoms, even still more preferably having 1 to 6 carbon atoms, and particularly preferably having 1 to 3 carbon atoms), an alkenyloxy group (preferably having 2 to 20 carbon atoms and more preferably having 2 to 12 carbon atoms), an alkynyloxy group (preferably having 2 to 20 carbon atoms and more preferably having 2 to 12 carbon atoms), a cycloalkyloxy group (preferably having 3 to 20 carbon atoms), an aryloxy group (preferably having 6 to 40 carbon atoms, more preferably having 6 to 26 carbon atoms, and still more preferably having 6 to 14 carbon atoms), a heterocyclic oxy group (preferably having 2 to 20 carbon atoms), a polyalkyleneoxy group,
an alkoxycarbonyl group (preferably having 2 to 20 carbon atoms), a cycloalkoxycarbonyl group (preferably having 4 to 20 carbon atoms), an aryloxycarbonyl group (preferably having 6 to 20 carbon atoms), an amino group (preferably having 0 to 20 carbon atoms, more preferably having 0 to 12 carbon atoms, still more preferably having 0 to 8 carbon atoms, even still more preferably having 0 to 6 carbon atoms, and particularly preferably having 0 to 3 carbon atoms; and including an unsubstituted amino group (-NH₂), as well as a mono- or di-substituted amino group substituted with a group selected from an alkyl group, an alkenyl group, an alkynyl group, a cycloalkyl group, a cycloalkenyl group, an aryl group, and a heterocyclic ring. The above-mentioned groups that substitute an unsubstituted amino group have the same definitions as the corresponding groups in the substituent group T.), a sulfamoyl group (preferably having 0 to 20 carbon atoms; and preferably an alkyl sulfamoyl group, a cycloalkyl sulfamoyl group, or an aryl sulfamoyl group), an acyl group (preferably having 1 to 20 carbon atoms and more preferably having 2 to 15 carbon atoms; and including -C(=O)H, an alkylcarbonyl group, a cycloalkylcarbonyl group, an arylcarbonyl group, and a heterocyclic carbonyl group), an acyloxy group (preferably having 1 to 20 carbon atoms), a carbamoyl group (preferably having 1 to 20 carbon atoms; and preferably an alkyl carbamoyl group, a cycloalkyl carbamoyl group, or an aryl carbamoyl group),
an acylamino group (preferably having 1 to 20 carbon atoms), a sulfonamide group (preferably having 0 to 20 carbon atoms; and preferably an alkyl sulfonamide group, a cycloalkyl sulfonamide group, or an aryl sulfonamide group), an alkylthio group (preferably having 1 to 20 carbon atoms and more preferably having 1 to 12 carbon atoms), a cycloalkylthio group (preferably having 3 to 20 carbon atoms), an arylthio group (preferably having 6 to 40 carbon atoms, more preferably having 6 to 26 carbon atoms, and still more preferably having 6 to 14 carbon atoms), a heterocyclic thio group (preferably having 2 to 20 carbon atoms), an alkyl, cycloalkyl, or aryl sulfonyl group (preferably having 1 to 20 carbon atoms),
a silyl group (preferably having 1 to 30 carbon atoms and more preferably 1 to 20 carbon atoms; preferably a silyl group in which an alkyl, aryl, alkoxy, or aryloxy has been substituted), a silyloxy group (preferably having 1 to 20 carbon atoms; preferably a silyloxy group in which an alkyl, aryl, alkoxy, or aryloxy has been substituted), a hydroxy group, a cyano group, a nitro group, a halogen atom (for example, a fluorine atom, a chlorine atom, a bromine atom, or an iodine atom), an oxygen atom (specifically, >CH₂ constituting a ring is replaced with >C=O), an anionic group (a carboxy group (-CO₂H), a phosphono group [-PO(OH)₂], a phosphonooxy group [-O-PO(OH)₂], or a sulfo group (-SO₃H)), a boric acid group [-B(OH)₂], a cationic group (also referred to as an onio group; including, an ammonio group containing a cyclic ammonio group, a sulfonio group, and a phosphonio group; and preferably having 0 to 30 carbon atoms and more preferably having 1 to 20 carbon atoms), a sulfanyl group (-SH), a guanidino group (-NHC(=NH)NH₂), a betaine residue, a polyol residue, a sugar residue, and a polyamino acid residue.

The preferred ranges of the anionic group, cationic group, betaine residue, polyol residue, sugar residue, polyamino acid residue, and polyalkyleneoxy group are as described above.

In addition, examples of groups obtained by combining a plurality of substituents selected from the substituent group T include the above-mentioned alkyl group, alkenyl group, alkynyl group, cycloalkyl group, cycloalkenyl group, aryl group, heterocyclic group, alkoxy group, alkenyloxy group, alkynyloxy group, cycloalkyloxy group, aryloxy group, heterocyclic oxy group, alkoxycarbonyl group, cycloalkoxycarbonyl group, aryloxycarbonyl group, amino group, sulfamoyl group, acyl group, acyloxy group, carbamoyl group, acylamino group, sulfonamide group, alkylthio group, cycloalkylthio group, arylthio group, heterocyclic thio group, and alkyl, cycloalkyl, or aryl sulfonyl group, each of which has, as a substituent, a substituent containing at least one of the groups selected from an anionic group, a cationic group, a betaine residue, a polyol residue, a sugar residue, a polyamino acid residue, -(O-L)_{g}R^{E}, or -(L-O)_{g}R^{E}.

The substituent selected from the substituent group T is more preferably an alkyl group, an alkenyl group, a cycloalkyl group, an aryl group, a heterocyclic group, an alkoxy group, a cycloalkoxy group, an aryloxy group, an acyl group, an alkoxycarbonyl group, a cycloalkoxycarbonyl group, an amino group, an acylamino group, a carbamoyl group, a cyano group, a halogen atom, an anionic group, a cationic group, a betaine residue, a polyol residue, a sugar residue, a polyamino acid residue, -(O-L)_{g}R^{E}, or -(L-O)_{g}R^{E}, and still more preferably an alkyl group, an alkenyl group, an aryl group, a heterocyclic group, an alkoxy group, an acyl group, an alkoxycarbonyl group, an amino group, an acylamino group, a carbamoyl group, a cyano group, an anionic group, a cationic group, a betaine residue, a polyol residue, a sugar residue, a polyamino acid residue, -(O-L)_{g}R^{E}, or -(L-O)_{g}R^{E}.

The substituents selected from the substituent group T include not only groups obtained by combining a plurality of substituents selected from the above-mentioned substituent group T, but also groups obtained by combining a plurality of the above-mentioned groups, unless otherwise specified. For example, in a case where a compound, a substituent, or the like contains an alkyl group, an alkenyl group, or the like, the alkyl group, the alkenyl group, or the like may be substituted or unsubstituted. In addition, in a case where a compound, a substituent, or the like contains an aryl group, a heterocyclic group, or the like, the aryl group, the heterocyclic group, or the like may be a monocyclic ring or a fused ring, and may be substituted or unsubstituted.

### Examples

Hereinafter, the present invention will be described in more detail with reference to Examples, but the present invention is not limited thereto. The room temperature means 25°C.

Hereinafter, the methods for synthesizing each compound will be described in more detail, but the starting materials, intermediates, and synthetic routes are not limited thereto.

The abbreviations used in the synthesis of each compound shown below are as follows.
SPPS: solid phase peptide synthesis
DBU: 1,8-diazabicyclo[5.4.0]-7-undecene
EDC: 1-ethyl-3-(3-dimethylaminopropyl)carbodiimide hydrochloride
NAC: N-acetylcysteine
PBS: phosphate-buffered saline
NMP: N-methyl-2-pyrrolidone
DMSO: dimethyl sulfoxide
DIPEA: N,N-diisopropylethylamine
DODT: 3,6-dioxa-1,8-octanedithiol
TFA: trifluoroacetic acid
TIPS: triisopropylsilane
Me: methyl group
Fmoc: 9-fluorenylmethyloxycarbonyl group
Pro: proline

In addition, %v/v means a percent by volume, and %w/v means a percent by weight by volume.

Unless otherwise specified, SNAP Ultra C18 (trade name, manufactured by Biotage, LLC) or Sfar C18 (trade name, manufactured by Biotage, LLC) was used as the carrier in reverse phase column chromatography, and Hi-Flash Column (trade name, manufactured by Yamazen Corporation) was used as the carrier in normal phase column chromatography.

The mixing ratio in the eluent used in reverse phase column chromatography or normal phase column chromatography is in terms of a volume ratio. For example, "acetonitrile:water = from 0:100 to 20:80" means that an eluent of "acetonitrile:water = 0:100" was changed to an eluent of "acetonitrile:water = 20:80".

Purification by liquid chromatography was carried out under the following conditions.
Column: XSelect CSH Prep C18 Column, particle size: 5 µm, OBD preparative column (inner diameter 19 mm × length 250 mm), manufactured by Waters Corporation
Column temperature: 40°C
Flow rate: 20 mL/min
Detection wavelengths: 220 nm, 254 nm
Solvent: liquid A: 0.1% formic acid-water
   liquid B: 0.1% formic acid-acetonitrile

The mass spectrum (MS) was measured using an ACQUITY SQD LC/MS System [manufactured by Waters Corporation, ionization method: electrospray ionization (ESI) method].

The retention time (RT) was measured using an ACQUITY SQD LC/MS System (manufactured by Waters Corporation) and shown in minutes (min).
Column: ACQUITY UPLC BEH C18 (particle size: 1.7 µm, inner diameter 2.1 mm × length 30 mm), manufactured by Waters Corporation
Solvent: liquid A: 0.1% formic acid-water
liquid B: 0.1% formic acid-acetonitrile
Gradient cycle: 0.00 min (liquid A/liquid B = 95/5), 2.00 min (liquid A/liquid B = 5/95), 3.00 min (liquid A/liquid B = 95/5)
Flow rate: 0.5 mL/min
Column temperature: room temperature
Detection wavelength: 254 nm

### (Compound (1): synthesis of (2S,4S)-1-(((9H-fluoren-9-yl)methoxy)carbonyl)-4-(3,5-bis(3-sulfopropoxy)benzamido)pyrrol idine-2-carboxylic acid)

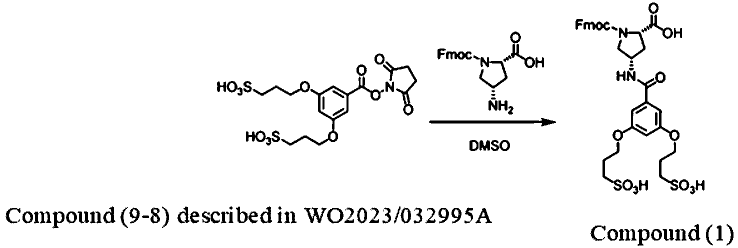

2.00 g of a compound (9-8) (that is, ((5-(((2,5-dioxopyrrolidin-1-yl)oxy)carbonyl)-1,3-phenylene)bis(oxy))bis(propane-1-sulfonic acid)) described in WO2023/032995A, which was synthesized based on the section of <Synthesis of compound (9)> described in WO2023/032995A, 10 mL of dimethyl sulfoxide (DMSO), 2.81 mL of triethylamine, and 1.42 g of (2S,4S)-4-amino-1-{[(9H-fluoren-9-yl)methoxy]carbonyllpyrrolidine-2-carboxylic acid (manufactured by Enamine Ltd.) were placed in a 50 mL eggplant flask which was then stirred at 50°C for 3 hours. After the reaction was completed, the reaction product was purified by reverse phase column chromatography and freeze-dried to obtain 1.25 g of a compound (1).

### (Compound (2): synthesis of (2S,4S)-1-(((9H-fluoren-9-yl)methoxy)carbonyl)-4-(4-(tert-butoxy)-4-oxobutanamido)pyrrolid ine-2-carboxylic acid)

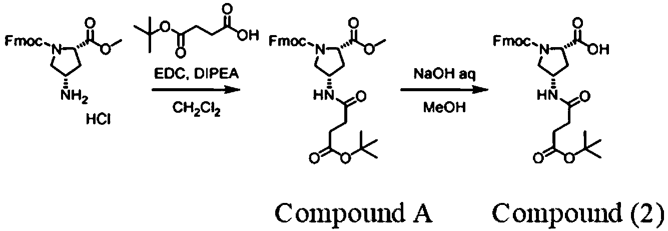

1.00 g of 1-(9H-fluoren-9-ylmethyl)-2-methyl-(2S,4S)-4-amino-1,2-pyrrolidine dicarboxylate hydrochloride, which was synthesized based on the section of <Preparation 5, Step 1> described in WO2018/020358A, 15 mL of dichloromethane, 562 mg of mono-tert-butyl succinate (manufactured by Tokyo Chemical Industry Co., Ltd.), 546 µL of N,N-diisopropylethylamine (manufactured by Tokyo Chemical Industry Co., Ltd.), and 600 mg of 1-ethyl-3-(3-dimethylaminopropyl)carbodiimide hydrochloride (manufactured by Dojindo Laboratories) were placed in a 50 mL eggplant flask which was then stirred at room temperature for 18 hours. After the reaction was completed, water and chloroform were added and the organic layer was separated. The organic layer was dried over sodium sulfate and filtered, and then the solvent was distilled off in vacuo to obtain 1.26 g of 1-((9H-fluoren-9-yl)methyl)-2-methyl-(2S,4S)-4-(4-(tert-butoxy)-4-oxobutanamido)pyrrolidin e-1,2-dicarboxylate (hereinafter, referred to as a "compound A").

Next, 1.26 g of the compound A was dissolved in 15 mL of methanol, and the solution was immersed in an ice bath. 7.2 mL of a 1 M aqueous sodium hydroxide solution was added thereto, and the mixture was stirred at room temperature for 1 hour. After the reaction was completed, 7.2 mL of a 1 M aqueous hydrochloric acid solution was added thereto, followed by extraction with ethyl acetate and then washing with saturated saline. The organic layer was dried over sodium sulfate and filtered, and the solvent was distilled off in vacuo. The residue was purified by silica gel chromatography (chloroform:methanol = 10:1) to obtain 924 mg of a compound (2).

### (Solid phase synthesis: synthesis of Pro linker)

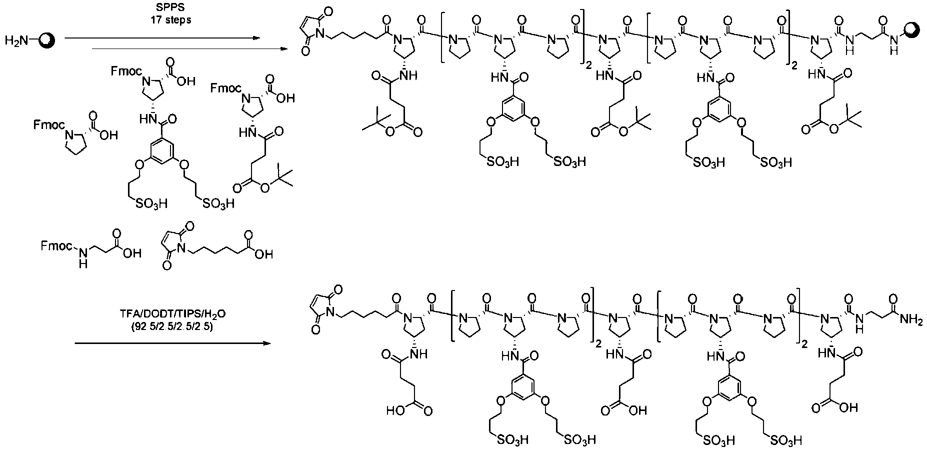

The solid phase peptide synthesis was carried out using an automated peptide synthesizer (trade name: Syro I, manufactured by Biotage, LLC). The synthesis of the peptide chain was carried out according to the general method of the solid phase peptide method described in WO2018/174078A.

A solid phase synthesis resin (H-Rink amide ChemMatrix resin (trade name, manufactured by Sigma-Aldrich Co., LLC)), an N-methyl-2-pyrrolidone (NMP) solution of Fmoc amino acid (0.5 M), an NMP solution of cyano-hydroxyimino-acetic acid ethyl ester (1 M) and N,N-diisopropylethylamine (0.1 M), an NMP solution of diisopropylcarbodiimide (1 M), an NMP solution of piperidine (20% v/v), and an NMP solution of acetic anhydride (20% v/v) were set in a synthesis device, and synthesis was carried out according to the manual. A cycle consisting of Fmoc deprotection (20 minutes), washing with NMP, condensation of Fmoc amino acids (1 hour), and washing with NMP as one cycle was repeated to elongate the peptide chain. After elongation of the peptide chain, the Fmoc group was deprotected, and 6-maleimidohexanoic acid was condensed in the same manner as with an amino acid.

Next, in order to cleave a linear peptide from the resin, a solution of trifluoroacetic acid:3,6-dioxa-1,8-octanedithiol:triisopropylsilane:water = 92.5:2.5:2.5:2.5 equivalent to a five-fold amount relative to the amount of the resin was added to the resin, and the mixture was shaken at room temperature for 2 hours. The reaction liquid was recovered by filtration. The reaction was repeated once more using a solution of trifluoroacetic acid:3,6-dioxa-1,8-octanedithiol:triisopropylsilane:water = 92.5:2.5:2.5:2.5, and the reaction liquid was recovered by filtration. All the recovered reaction liquids were combined, the solvent was distilled off under reduced pressure, and the residue was thoroughly dried to obtain a crude purified product of a linear peptide.

The purification of the crude purified product obtained was carried out by liquid chromatography. The fraction of the target substance was collected, and the solvent was distilled off in vacuo to obtain a target Pro linker described in the above scheme as a freeze-dried powder.
m/z (z = 2): [M (target Pro linker) + 2H⁺]²⁺/2 = 1833

The Fmoc-amino acid was used which was obtained from Watanabe Chemical Industries, Ltd.

N-methyl-2-pyrrolidone, N,N-diisopropylethylamine, diisopropylcarbodiimide, piperidine, and acetic anhydride were obtained from FUJIFILM Wako Pure Chemical Corporation.

The cyano-hydroxyimino-acetic acid ethyl ester and the 6-maleimidohexanoic acid were obtained from Tokyo Chemical Industry Co., Ltd.

### (Synthesis of payload-degradable linker)

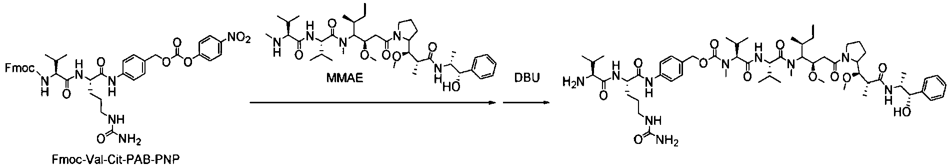

267 mg of monomethyl auristatin E (MMAE, manufactured by Selleck Biotechnology Limited) and 300 mg of Fmoc-Val-Cit-PAB-PNP (manufactured by Tokyo Chemical Industry Co., Ltd., a dipeptide linker suitable for ADC) were dissolved in 3 mL of dimethylformamide in a 10 mL eggplant flask which was then stirred at room temperature for 18 hours. Next, the reaction liquid was poured into 50 mL of water to obtain a precipitate. The precipitate was collected by filtration, washed with water, and then air-dried at room temperature overnight to obtain a white solid.

Next, the obtained white solid was dissolved in 3 mL of dimethylformamide, and 59 µL of 1,8-diazabicyclo[5.4.0]undec-7-ene (DBU) was added thereto, followed by stirring at room temperature for 1 hour.

The solution after the reaction was purified by liquid chromatography. The fraction of the target substance was collected, and the solvent was distilled off in vacuo to obtain a target payload-degradable linker described in the above scheme as a freeze-dried powder.
m/z (z = 1): [M (target payload-degradable linker) + H⁺]⁺ = 1124

### (Synthesis of drug linker)

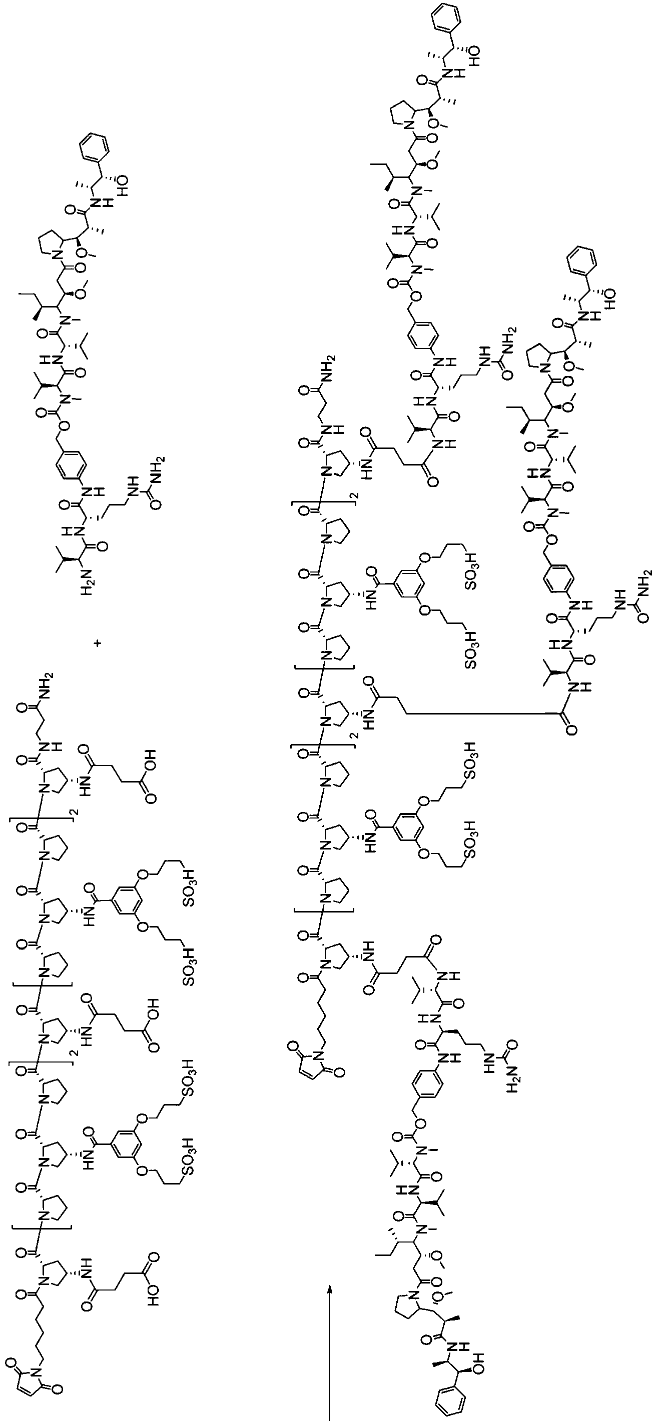

100 mg of the Pro linker synthesized above and 92 mg of the payload-degradable linker synthesized above were placed in a 10 mL eggplant flask and dissolved in 1 mL of dimethylformamide. 17 g (3.3 equivalents) of 1-ethyl-3-(3-dimethylaminopropyl)carbodiimide hydrochloride (manufactured by Dojindo Laboratories) was added thereto, followed by stirring at room temperature for 18 hours.

The solution after the reaction was purified by liquid chromatography. The fraction of the target substance was collected, and the solvent was distilled off in vacuo to obtain a target drug linker described in the above scheme as a freeze-dried powder.
m/z (z = 4): [M (target drug linker) + 4H⁺]⁴⁺/4 = 1746

### (Synthesis of ADC)

An antibody drug conjugate (ADC) was synthesized as follows, with reference to the synthesis of an antibody drug conjugate described in WO2015/115091A.

### (1) Reduction of antibody

10 mg of a PBS solution of trastuzumab (molecular weight: approximately 148000) (concentration 20 mg/mL, a PBS solution of an antibody that specifically binds to the "HER2 protein" present on the surface of a cancer cell, manufactured by Biosynth AG) was placed in a 1.5 mL polypropylene tube and diluted with 0.5 mL of D-PBS (manufactured by FUJIFILM Wako Pure Chemical Corporation) (concentration: 10 mg/mL). 11 µL of tris(2-carboxyethyl)phosphine hydrochloride (TCEP, manufactured by FUJIFILM Wako Pure Chemical Corporation) adjusted to a concentration of 10 mM (1.65 equivalents with respect to one antibody molecule) was added thereto, and it was confirmed that the pH of the prepared aqueous solution was within 7.4 ± 0.1. This was followed by incubation at 37°C for 1 hour to reduce the disulfide bond within the antibody.

### (2) Synthesis of antibody drug conjugate linker conjugate

The polypropylene tube containing the above solution was immersed in a water bath at 25°C and allowed to stand for 10 minutes, and then 20 µL of a 10 mM drug linker (3 equivalents with respect to one antibody molecule) dissolved in DMSO was added thereto, and the tube was incubated for 4 hours while still immersed in the water bath at 25°C, allowing the drug linker to bind to the antibody.

Next, 81 µL of an aqueous solution of N-acetylcysteine (NAC, manufactured by FUJIFILM Wako Pure Chemical Corporation) adjusted to a concentration of 10 mM (12 equivalents with respect to one antibody molecule) was added thereto, followed by further incubation at 25°C for 20 minutes to terminate the reaction of the drug linker, thereby obtaining a reaction liquid of an antibody drug conjugate.

### (3) Purification

The purification was carried out with reference to the description relating to the purification of an antibody drug conjugate described in paragraph [0110] of WO2015/115091A.

1.5 mL of the above-mentioned antibody drug conjugate reaction liquid was placed on a NAP-25 column (Cat. No. 17-0852-02, manufactured by GE Healthcare Japan Corporation) using Sephadex G-25 carrier equilibrated with D-PBS (manufactured by FUJIFILM Wako Pure Chemical Corporation), and elution was carried out according to the procedure attached to the NAP-25 to recover the target substance fraction. This preparative fraction was purified by repeating the process three times with NAP-25 to obtain a target antibody drug conjugate (ADC).

The target antibody drug conjugate (ADC) obtained as described above is mainly an antibody drug conjugate having a structure in which six drugs (payload, MMAE, manufactured by Selleck Biotechnology Limited) having high hydrophobicity are bound to one molecule of an antibody having high hydrophilicity. In addition, a solution of this antibody drug conjugate (ADC) at a concentration of 0.5% by mass in D-PBS (manufactured by FUJIFILM Wako Pure Chemical Corporation) was present as a homogeneous solution without any aggregation, confirming that aggregation of the antibody drug conjugate (ADC) was suppressed. The above-mentioned antibody drug conjugate (ADC) does not undergo aggregation at all in a structure in which the number of drugs that bind to one antibody molecule is 6, so it can be understood that aggregation of the antibody drug conjugate (ADC) according to the embodiment of the present invention can be sufficiently suppressed even in a case where a structure in which the number of drugs that bind to one antibody molecule is equal to or more than 6 (for example, 8 or more) is adopted.

This application claims priority based on JP2023-141861 filed on August 31, 2023, the contents of which are incorporated herein by reference as part of the present specification.

## Claims

1. A compound or a salt thereof, comprising:
two or more structural moieties M selected from a physiologically active substance moiety and a radioisotope-containing moiety; and
a structure represented by General Formula (I),
wherein the compound and a salt do not have a phosphor moiety and have at least one group selected from the following hydrophilic group Pi, 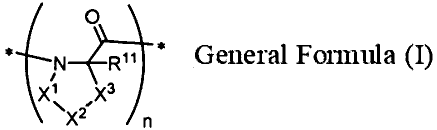
in the formula, X¹ to X³ each represent -O-, -S-, >NR¹, or >CR²R³,
R¹ to R³ and R¹¹ each represent a hydrogen atom, an alkyl group, an alkenyl group, an alkynyl group, an aryl group, a heteroaryl group, an acyl group, -NR⁸R⁹, -OR¹⁰, or a group selected from the following hydrophilic group Pi,
R⁸ to R¹⁰ each represent a hydrogen atom, an alkyl group, an alkenyl group, an alkynyl group, an acyl group, an aryl group, a heteroaryl group, or a group selected from the following hydrophilic group Pi,
n is an integer of 2 or more, and
* represents a bonding site,
<Hydrophilic group Pi>
an anionic group, a cationic group, a betaine residue, a polyol residue, a sugar residue, a polyamino acid residue, and a polyalkyleneoxy group.

2. The compound or a salt thereof according to claim 1, which is represented by General Formula (II), 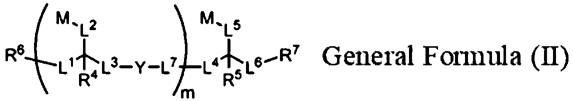
in the formula, R⁴ and R⁵ each represent a hydrogen atom, an alkyl group, an alkenyl group, an alkynyl group, an acyl group, an amino group, a hydroxy group, an alkoxy group, a sulfanyl group, an aryl group, or a heteroaryl group,
R⁶ and R⁷ each represent a hydrogen atom, a hydroxy group, a sulfanyl group, an alkyl group, an alkenyl group, an alkynyl group, an aryl group, an alkoxy group, an amino group, an acyl group, a heteroaryl group, or Q, provided that at least one of R⁶ or R⁷ is a group containing a carboxy group or a group containing a substituent capable of binding to a target binding substance,
Q represents a group selected from the hydrophilic group Pi or a substituent capable of binding to the target binding substance,
L¹, L³, L⁴, L⁶, and L⁷ each represent a single bond or a linking group,
L² and L⁵ each represent a single bond or a physiologically cleavable linker,
provided that, in a case where L² is a single bond, at least one of L¹ or L³ is a physiologically cleavable linker, and in a case where L⁵ is a single bond, at least one of L⁴ or L⁶ is a physiologically cleavable linker,
M represents the structural moiety M,
Y represents the structure represented by General Formula (I), and
m is an integer of 1 or more.

3. The compound or a salt thereof according to claim 2, which is represented by General Formula (III), 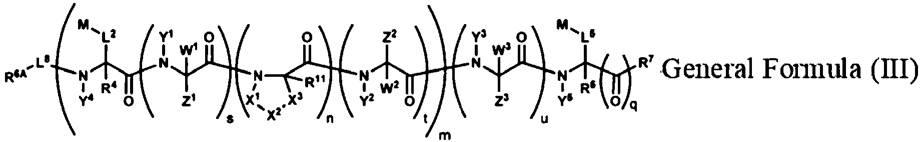
in the formula, Y¹ to Y⁵, Z¹ to Z³, and W¹ to W³ each represent a hydrogen atom, an alkyl group, an alkenyl group, an alkynyl group, an acyl group, an amino group, a hydroxy group, an alkoxy group, a sulfanyl group, an aryl group, a heteroaryl group, or a group selected from the hydrophilic group Pi,
R^{6A} represents a hydrogen atom, a hydroxy group, a sulfanyl group, an alkyl group, an alkenyl group, an alkynyl group, an aryl group, an alkoxy group, an amino group, an acyl group, a heteroaryl group, or Q, provided that R^{6A} is a carboxy group or a substituent capable of binding to a target binding substance and/or R⁷ is a group containing a carboxy group or a group containing a substituent capable of binding to the target binding substance,
R⁴, R⁵, R⁷, R¹¹, L², L⁵, X¹ to X³, M, Q, m, and n have the same definitions as R⁴, R⁵, R⁷, R¹¹, L², L⁵, X¹ to X³, M, Q, m, and n described above,
L⁸ represents a single bond or a linking group,
s, t, and u each represent 0 or an integer of 1 or more, and
q is an integer of 0 or 1.

4. The compound or a salt thereof according to claim 1, which is represented by General Formula (IV), 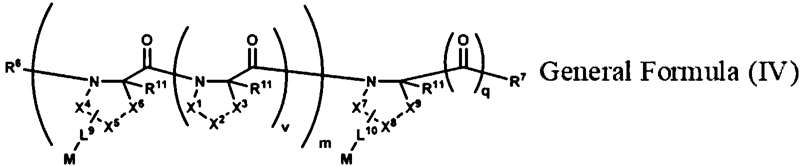
in the formula, X⁴ to X⁹ each represent -O-, -S-, >NR¹, or >CR²R³,
provided that at least one of X⁴, X⁵, or X⁶ is >N-L⁹-M or >C(R²)-L⁹-M, and at least one of X⁷, X⁸, or X⁹ is >N-L¹⁰-M or >C(R²)-L¹⁰-M,
R¹ to R³ which are neither -L⁹-M nor -L¹⁰-M have the same definitions as R¹ to R³ described above,
L⁹ and L¹⁰ each represent a physiologically cleavable linker,
R⁶ and R⁷ each represent a hydrogen atom, a hydroxy group, a sulfanyl group, an alkyl group, an alkenyl group, an alkynyl group, an aryl group, an alkoxy group, an amino group, an acyl group, a heteroaryl group, or Q, provided that at least one of R⁶ or R⁷ is a group containing a carboxy group or a group containing a substituent capable of binding to a target binding substance,
Q represents a group selected from the hydrophilic group Pi or a substituent capable of binding to the target binding substance,
m is an integer of 1 or more, q is an integer of 0 or 1, and v is 0 or an integer of 1 or more, and
R¹¹, X¹ to X³, and M have the same definitions as R¹¹, X¹ to X³, and M described above.

5. The compound or a salt thereof according to claim 4, which is represented by General Formula (V), 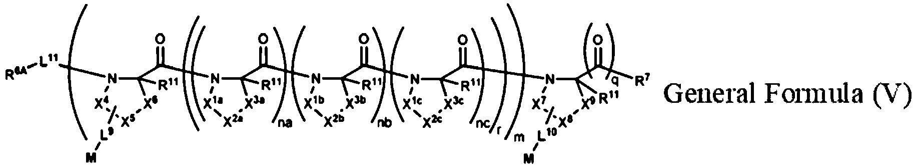
in the formula, X^{1a}, X^{1b}, X^{1c}, X^{2a}, X^{2b}, X^{2c}, X^{3a}, X^{3b}, and X^{3c} each represent -O-, -S-, >NR¹, or >CR²R³,
L¹¹ represents a single bond or a linking group,
R^{6A} represents a hydrogen atom, a hydroxy group, a sulfanyl group, an alkyl group, an alkenyl group, an alkynyl group, an aryl group, an alkoxy group, an amino group, an acyl group, a heteroaryl group, or Q, provided that R^{6A} is a carboxy group or a substituent capable of binding to a target binding substance and/or R⁷ is a group containing a carboxy group or a group containing a substituent capable of binding to the target binding substance,
R¹ to R³, R⁷, R¹¹, X⁴ to X⁹, L⁹, L¹⁰, M, Q, m, and q have the same definitions as R¹ to R³, R⁷, R¹¹, X⁴ to X⁹, L⁹, L¹⁰, M, Q, m, and q described above,
na, nb, and nc are each 0 or an integer of 1 or more, and r is an integer of 1 or more.

6. The compound or a salt thereof according to claim 1, which has three to six structural moieties M.

7. A targeted drug conjugate comprising:
the compound or a salt thereof according to claim 1; and
a target binding substance,
wherein the compound or a salt thereof is bound to the target binding substance.

8. The targeted drug conjugate according to claim 7,
wherein the target binding substance is an antibody.

9. The targeted drug conjugate according to claim 8,
wherein the number of the structural moieties M in one molecule of the targeted drug conjugate is 8 or more.

10. A medicine comprising:
the targeted drug conjugate according to claim 7.
